# EUROPEAN PATENT SPECIFICATION

(11) **EP 1 659 855 B1**
(45) Date of publication and mention of the grant of the patent: **02.11.2011**
(21) Application number: 04786222.2
(22) Date of filing: 30.08.2004
(51) Int. Cl.: A01H 5/00, C12N 15/29, C12N 15/60, C12N 9/88

(54) **RICE PLANTS HAVING INCREASED TOLERANCE TO IMIDAZOLINONE HERBICIDES**
REISPFLANZEN MIT ERHÖHTER TOLERANZ GEGEN IMIDAZOLINON-HERBIZIDE
PLANTS DE RIZ PRESENTANT UNE TOLERANCE ACCRUE AUX HERBICIDES IMIDAZOLINONE

(30) Priority: 29.08.2003 US 498895 P; 30.12.2003 US 533105 P
(43) Date of publication of application: 31.05.2006
(60) Divisional application: 10180406.0
(73) Proprietor: Instituto Nacional de Tecnologia Agropecuaria, 01033 Buenos Aires (AR)
(72) Inventor: LIVORE, Alberto B., 3260 Entre Rios (AR); PRINA, Alberto R., 6600 Pcia. De Buenos Aires (AR); BIRK, Iwona, Raleigh, NC 27613 (US); SINGH, Bijay, Cary, NC 27519 (US)
(74) Representative: Reitstötter - Kinzebach
(86) International application number: PCT/EP2004/009641
(87) International publication number: WO 2005/020673

(56) References cited:
- EP-A2- 0 461 355
- WO-A1-01/82685
- WO-A2-01/85970
- US-A- 5 013 659
- US-A1- 2003 097 692

## Description

### Field of the Invention

The present invention relates in general to plants having an increased tolerance to imidazolinone herbicides. More specifically, the present invention relates to rice plants obtained by mutagenesis and cross-breeding and transformation that have an increased tolerance to imidazolinone herbicides.

### Background of the Invention

According to a farmer's survey, the major constraints to rice production are weeds (Hidaka et al., Agrochemicals Japan, 2000, 77: 21-29). Direct seeding has reduced the labor problems of transplanting, however this technology has helped to increase the weed problem. Herbicide use in rice crops is a common practice in most of the rice regions that direct seed rice crops and/or in developed countries that grow rice under either transplanting or direct seeding systems. Usually a grass and a broadleaf herbicide are applied one or more times in order to control weeds in rice crops.

Grasses, sedges and weedy rice ("red rice") have been the major groups of species that possess high *fitness to the same environments where rice is grown.* These weeds have become globally distributed and are difficult to control in rice crops. Red rice belongs to the same species as cultivated rice (*Oryza sativa L*). The genetic similarity of red rice and commercial rice has made herbicidal control of red rice difficult. Several cultural practices aid in control of weeds and are convenient for better environmental care, such as land preparation, land leveling, levees and depth of water, land rotation, certified seed, proper plant systems and dates of planting. Although these cultural practices may help to reduce the weed seed bank and the development of herbicide tolerant weeds, they impose certain restrictions and increase the cost of the crop.

In spite of the many recommendations for better cultural practices, farmers still rely on the use of herbicides as the main tool to control weeds. The use and abuse of some of these chemicals has resulted in the development of tolerant weeds like Propanil and Butachlor resistant bamyardgrass (*Echinochloa crus galli*). In these cases, it is convenient to have other herbicides with different modes of action with the ability to control most of these weed species, so that their application can be alternated with the herbicides commonly applied.

Acetohydroxyacid synthase (AHAS; EC 4.1.3.18, acetolactate synthase (ALS)), encoded by the AHAS nucleic acid, is the first enzyme that catalyzes the biochemical synthesis of the branched chain amino acids valine, leucine, and isoleucine (Singh B. K., 1999, Biosynthesis of valine, leucine and isoleucine in: Singh B. K. (Ed) Plant amino acids. Marcel Dekker Inc. New York, New York. Pg 227-247). AHAS is the site of action of four structurally diverse herbicide families including the sulfonylureas (LaRossa RA and Falco SC, 1984, Trends Biotechnol. 2:158-161), the imidazolinones (Shaner et al., 1984, Plant Physiol. 76:545-546), the triazolopyrimidines (Subramanian and Gerwick, 1989, Inhibition of acetolactate synthase by triazolopyrimidines in (Ed) Whitaker JR, Sonnet PE Biocatalysis in agricultural biotechnology. ACS Symposium Series, American Chemical Society. Washington, D.C. Pg 277-288), and the pyrimidyloxybenzoates (Subramanian et al., 1990, Plant Physiol. 94: 239-244.). Imidazolinone and sulfonylurea herbicides are widely used in modern agriculture due to their effectiveness at very low application rates and relative non-toxicity in animals. By inhibiting AHAS activity, these families of herbicides prevent further growth and development of susceptible plants including many weed species. Several examples of commercially available imidazolinone herbicides are PURSUIT® (imazethapyr), SCEPTER® (imazaquin) and ARSENAL® (imazapyr). Examples of sulfonylurea herbicides are chlorsulfuron, metsulfuron methyl, sulfometuron methyl, chlorimuron ethyl, thifensulfuron methyl, tribenuron methyl, bensulfuron methyl, nicosulfuron, ethametsulfuron methyl, rimsulfuron, triflusulfuron methyl, triasulfuron, primisulfuron methyl, cinosulfuron, amidosulfuron, fluzasulfuron, imazosulfuron, pyrazosulfuron ethyl, and halosulfuron.

Due to their high effectiveness and low toxicity, imidazolinone herbicides are favored for application by spraying over the top of a wide area of vegetation. The ability to spray an herbicide over the top of a wide range of vegetation decreases the costs associated with plantation establishment and maintenance, and decreases the need for site preparation prior to use of such chemicals. Spraying over the top of a desired tolerant species also results in the ability to achieve maximum yield potential of the desired species due to the absence of competitive species. However, the ability to use such spray-over techniques is dependent upon the presence of imidazolinone tolerant species of the desired vegetation in the spray over area.

Among the major agricultural crops, some leguminous species such as soybean are naturally resistant to imidazolinone herbicides due to their ability to rapidly metabolize the herbicide compounds (Shaner and Robson, 1985, Weed Sci. 33:469-471). Other crops such as corn (Newhouse et al., 1992, Plant Physiol. 100:882-886) and rice (Barrett et al., 1989, Crop Safeners for Herbicides, Academic Press New York, pp. 195-220) are susceptible to imidazolinone herbicides. The differential sensitivity to the imidazolinone herbicides is dependent on the chemical nature of the particular herbicide and differential metabolism of the compound from a toxic to a non-toxic form in each plant (Shaner et al., 1984, Plant Physiol. 76:545-546; Brown et al., 1987, Pestic. Biochem. Physiol. 27:24-29). Other plant physiological differences such as absorption and translocation also play an important role in sensitivity (Shaner and Robson, 1985, Weed Sci. 33:469-471).

Crop cultivars resistant to imidazolinones, sulfonylureas and triazolopyrimidines have been successfully produced using seed, microspore, pollen, and callus mutagenesis in *Zea mays, Brassica napus, Glycine max,* and *Nicotiana tabacum* (Sebastian et al., 1989, Crop Sci. 29:1403-1408; Swanson ef al., 1989, Theor. Appl. Genet. 78:525-530; Newhouse et al., 1991, Theor. Appl. Genet. 83:65-70; Sathasivan et al., 1991, Plant Physiol. 97:1044-1050; Mourand ef al., 1993, J. Heredity 84:91-96). In all cases, a single, partially dominant nuclear gene conferred resistance. Four imidazolinone resistant wheat plants were also previously isolated following seed mutagenesis of *Triticum aestivum* L. cv Fidel (Newhouse et al., 1992, Plant Physiol. 100:882-886). Inheritance studies confirmed that a single, partially dominant gene conferred resistance. Based on allelic studies, the authors concluded that the mutations in the four identified lines were located at the same locus. One of the Fidel cultivar resistance genes was designated FS-4 (Newhouse et al., 1992, Plant Physiol. 100:882-886).

Computer-based modeling of the three dimensional conformation of the AHAS-inhibitor complex predicts several amino acids in the proposed inhibitor binding pocket as sites where induced mutations would likely confer selective resistance to imidazolinones (Ott et al., 1996, J. Mol. Biol. 263:359-368). Tobacco plants produced with some of these rationally designed mutations in the proposed binding sites of the AHAS enzyme have in fact exhibited specific resistance to a single class of herbicides (Ott et al., 1996, J. Mol. Biol. 263:359-368).

Plant resistance to imidazolinone herbicides has also been reported in a number of patents. U.S. Patent Nos. 4,761,373, 5,331,107, 5,304,732, 6,211,438, 6,211,439, and 6,222,100 generally describe the use of an altered AHAS nucleic acid to elicit herbicide resistance in plants, and specifically disclose certain imidazolinone resistant com lines. U.S. Patent No. 5,013,659 discloses plants exhibiting herbicide resistance possessing mutations in at least one amino acid in one or more conserved regions. The mutations described therein encode either cross-resistance for imidazolinones and sulfonylureas or sulfonylurea-specific resistance, but imidazolinone-specific resistance is not described. Additionally, U.S. Patent No. 5,731,180 and U.S. Patent No. 5,767,361 discuss an isolated gene having a single amino acid substitution in a wild-type monocot AHAS amino acid sequence that results in imidazolinone-specific resistance.

Transgenic and herbicide resistant rice plants have also been described. A rice mutant resistant to a sulfonylurea herbicide, derived by selective pressure on callus tissue culture was described, where resistance was attributed to a mutant AHAS enzyme (Terakawa et al., "Rice Mutant Resistant to the Herbicide Bensulfuron Methyl (BSM) by in vitro Selection," Japan. J. Breed., 1992 vol. 42:267-275). Other herbicide resistant rice plant varieties have been described in patents and patent applications, including WO 97/41218, WO 01/85970 and U.S. Pat. Nos. 5,545,822, 5,736,629, 5,773,704, U.S. Pat. No. 5,773,703, 5,952,553, and 6,274,796. U.S. Pat. No. 5,545,822 discloses a line of rice plants having a metabolically-based resistance to herbicides that interfere with the plant enzyme acetohydroxyacid synthase; i.e., the herbicide resistance of these rice plants was not due to a resistant AHAS enzyme. WO 97/41218 discloses one line of rice plants having a variant AHAS enzyme that is resistant to herbicides that interfere with the wild-type plant enzyme acetohydroxyacid synthase. This line of rice plants was developed by exposing rice seeds to the mutagen methanesulfonic acid ethyl ester (EMS), and screening millions of progeny for herbicide resistance.

What is needed in the art is the identification of further rice lines comprising imidazolinone resistance genes. What are also needed in the art are rice plants having increased tolerance to herbicides such as imidazolinone and containing at least one altered AHAS nucleic add. Also needed are methods for controlling weed growth in the vicinity of such rice plants. These compositions and methods would allow for the use of spray over techniques when applying herbicides to areas containing rice plants.

### Summary of the invention

The present invention provides rice plants comprising non-recombinant variant AHAS nucleic acids, wherein the variant nucleic acid confers upon the rice plant increase tolerance to an imidazolinone herbicides as compared to a wild-type variety of the plant, wherein the rice plant is a derivative or progeny of the rice plant with Patent Deposit Designation Number NCIMB 41206, NCIMB 41207, or NCIMB 41208, wherein the rice plant with Patent Deposit Designation Number NCIMB 41206, NCIMB 41207, or NCIMB 41208 comprises the variant AHAS nucleic acid. The variant AHAS nucleic acid encodes a variant AHAS protein comprising an alanine to threonine substitution as compared to a wild-type AHAS protein. Also provided are plant parts and plant seeds derived from the rice plants described herein.

The variant AHAS nucleic acids of the present invention comprise a polynucleotide sequence selected from the group consisting of: a polynucleotide as defined in SEQ ID NO:1; a polynucleotide as defined in SEQ ID NO:3; a polynucleotide as defined in SEQ ID NO:5; a polynucleotide as defined in SEQ ID NO:11; a polynucleotide sequence encoding a polypeptide as defined in SEQ ID NO:2; a polynucleotide sequence encoding a polypeptide as defined in SEQ ID NO:4; a polynucleotide sequence encoding a polypeptide as defined in SEQ ID NO:6; a polynucleotide sequence encoding a polypeptide as defined in SEQ ID NO:12; and a polynucleotide complementary to any of the aforementioned polynucleotides, wherein the variant AHAS nucleic acid encodes an AHAS polypeptide conferring increased tolerance to an imidazolinone herbicide as compared to a wild-type AHAS polypeptide.

The plants of the present invention can be transgenic or non-transgenic. In one embodiment, the plants of the present invention are non-transgenlc. Example of non-transgenic rice plants having increased tolerance to imidazolinone herbicides include a rice plant having NCIMB Patent Deposit Designation Number NCIMB 41208, NCIMB 41207, or NCIMB 41208; or a mutant, recombinant, or genetically engineered derivative of the plant with NCIMB Patent Deposit Designation Number NCIMB 41206, NCIMB 41207, or NCIMB 41208; or any progeny of the plant with NCIMB Patent Deposit Designation Number NCIMB 41206, NCIMB 41207, or NCIMB 41208; or a plant that is a progeny of any of these plants.

In addition to the compositions of the present invention, several methods are provided. Described herein are methods of modifying a rice plant's tolerance to an imidazolinone herbicide comprising modifying the expression of an AHAS nucleic acid in the plant. Also described are methods of producing a transgenic plant having increased tolerance to an imidazolinone herbicide comprising, transforming a plant cell with an expression vector comprising one or more variant AHAS nucleic acids encoding a variant AHAS protein comprising an alanine to threonine substitution as compared to a wild-type AHAS protein and generating the plant from the plant cell. The invention further includes a method of controlling weeds within the vicinity of a rice plant, comprising applying an imidazolinone herbicide to the weeds and to the rice plant, wherein the rice plant has increased tolerance to the imidazolinone herbicide as compared to a wild-type variety of the rice plant and wherein the plant comprises one or more AHAS nucleic acids encoding a variant AHAS protein comprising an alanine to threonine substitution as compared to a wild-type AHAS protein.

### BRIEF DESCRIPTION OF THE DRAWINGS

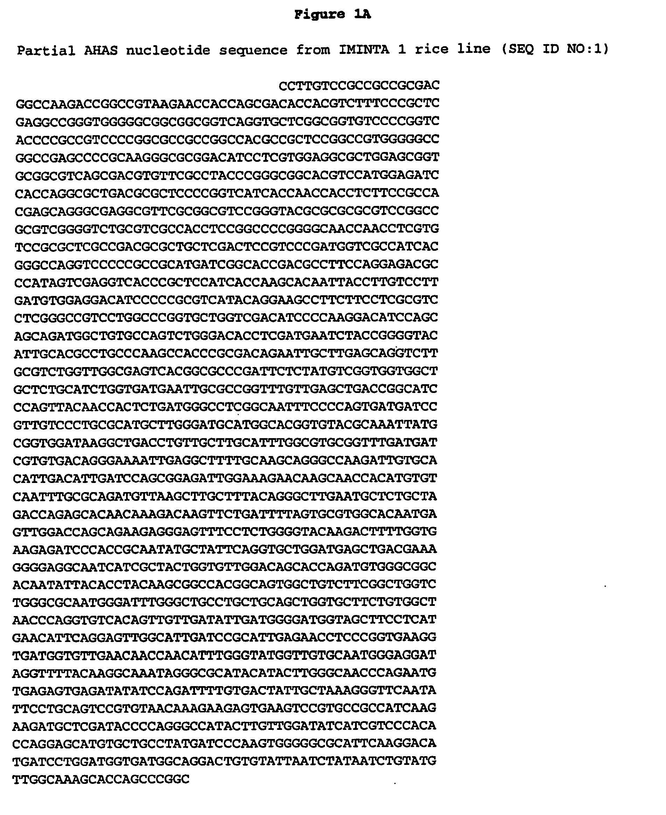

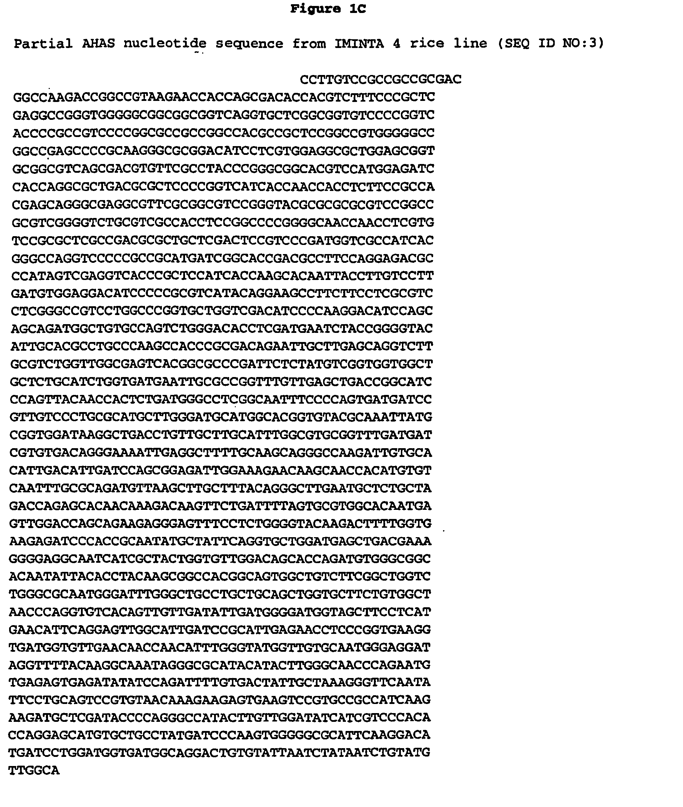

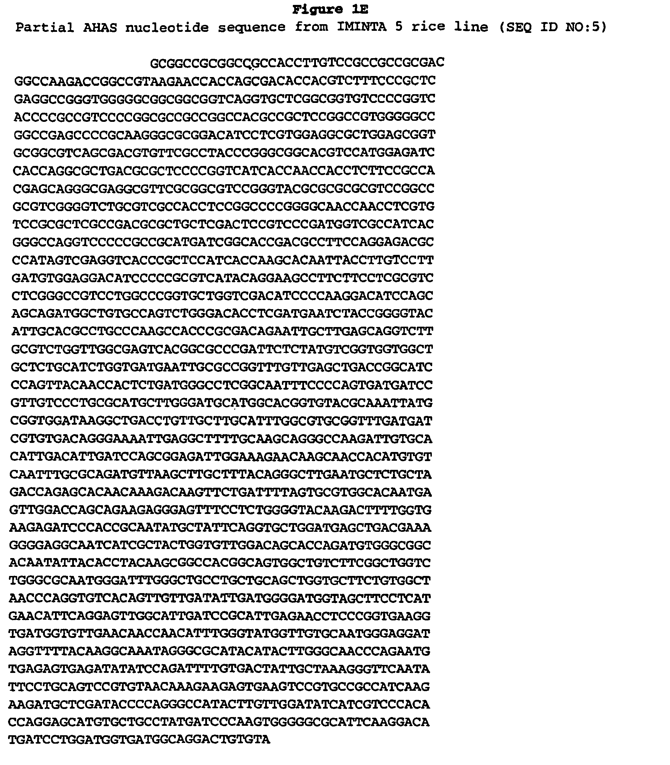

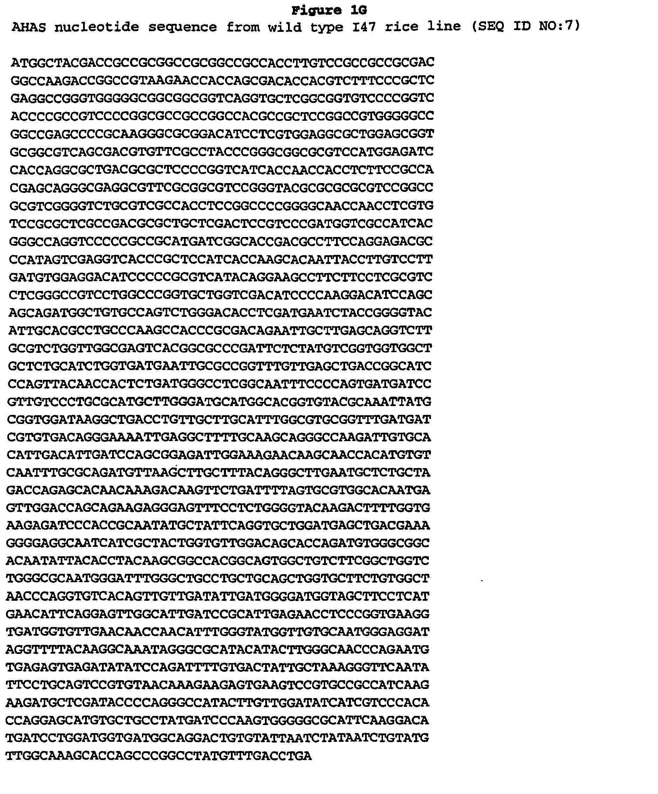

Figures 1A-B show the partial cDNA sequence of the IMINTA 1 AHAS nucleic acid (SEQ ID NO:1) and the deduced amino acid sequence thereof (SEQ ID NO:2). Figures 1C-D show the partial cDNA sequence of the IMINTA 4 AHAS nucleic acid (SEQ ID NO:3) and the deduced amino acid sequence thereof (SEQ ID NO:4). Figures 1E-F show the partial cDNA sequence of the IMINTA 5 AHAS nucleic acid (SEQ ID NO:5) and the deduced amino acid sequence thereof (SEQ ID NO:6). Figures 1G-H show the partial cDNA sequence of the wild-type IRGA 417 AHAS nucleic acid (SEQ ID NO:7) and the deduced amino acid sequence thereof (SEQ ID NO:8).

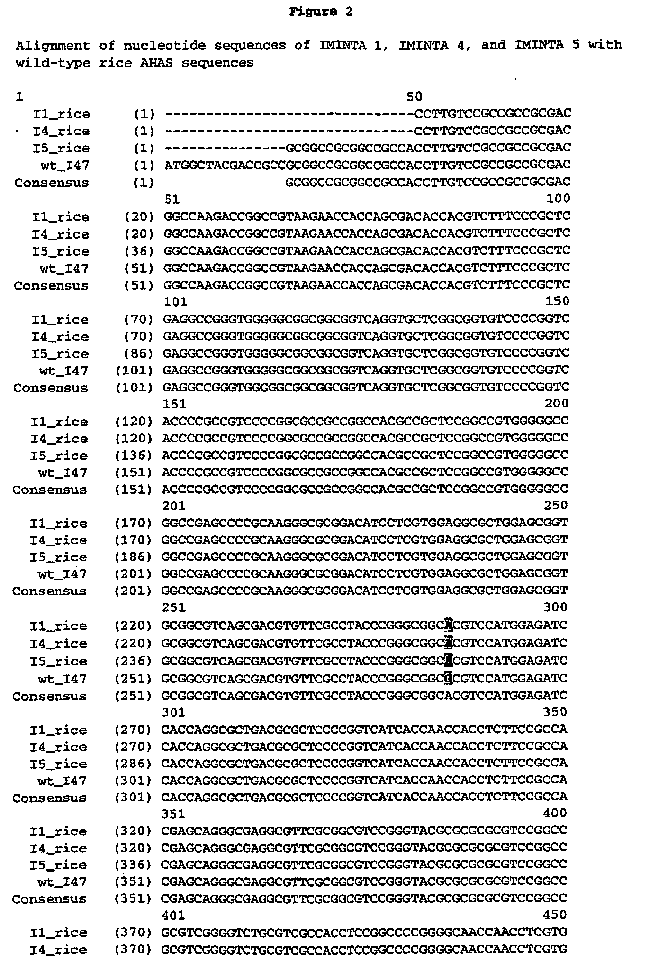

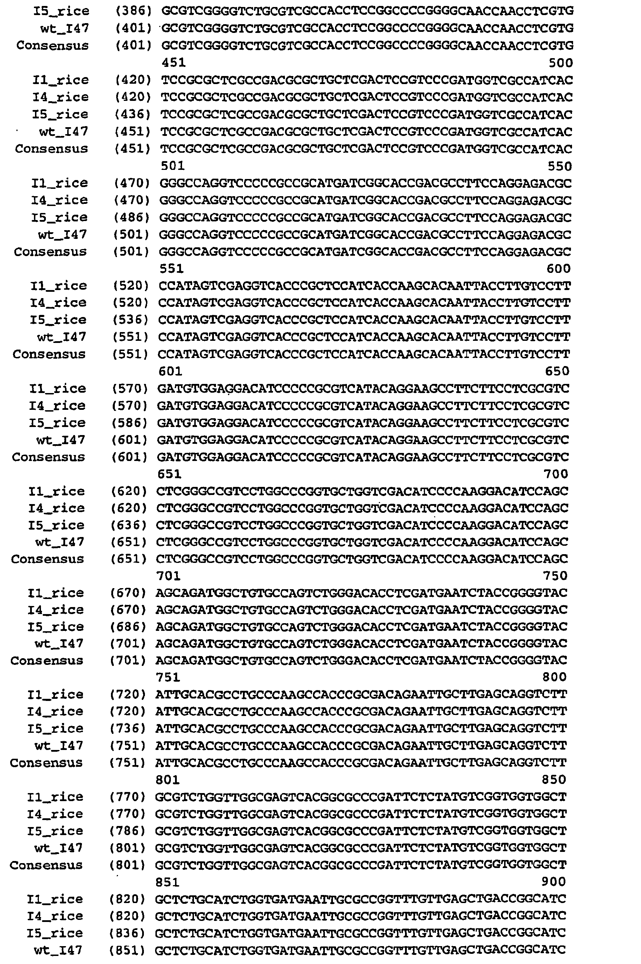

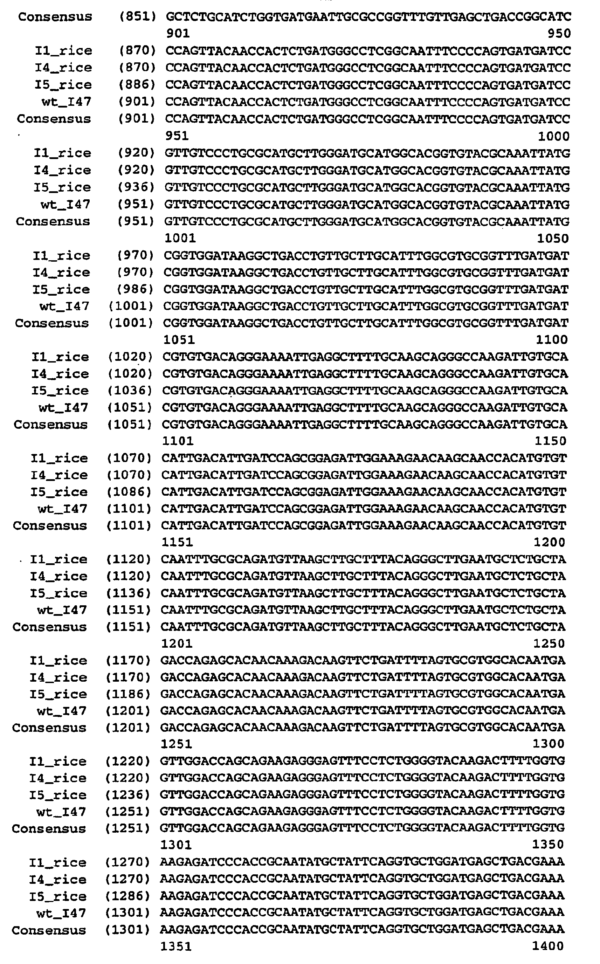

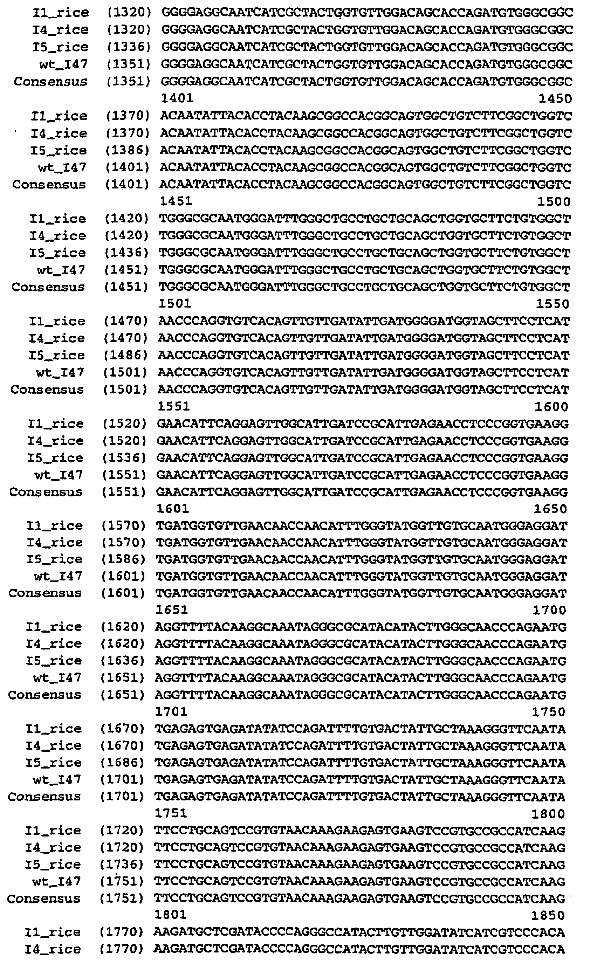

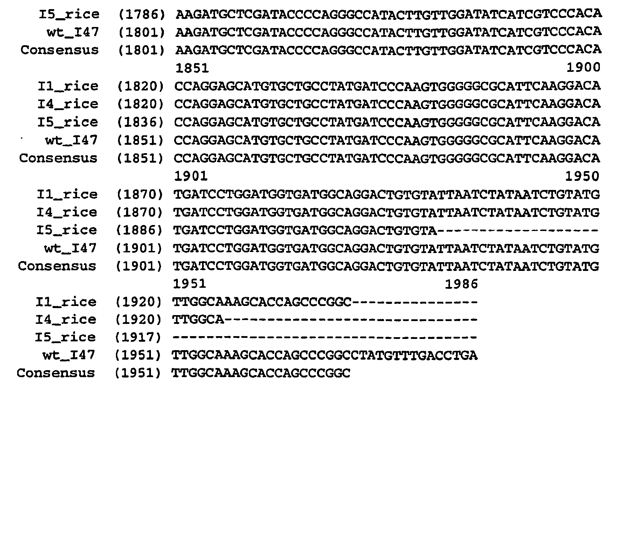

Figure 2 shows the cDNA sequence alignment of the AHAS gene amplified from genomic DNA from the imidazolinone tolerant IMINTA 1 line (SEQ ID NO:1), the AHAS gene amplified from genomic DNA from the imidazolinone tolerant IMINTA 4 line (SEQ ID NO:3), the AHAS gene amplified from genomic DNA from the imidazolinone tolerant IMINTA 5 line (SEQ ID NO:5), the AHAS gene amplified from genomic DNA from the IRGA 417 wild-type rice line (SEQ ID NO:7), and a rice AHAS gene consensus sequence (SEQ ID NO:9). The nucleotide polymorphism conferring the imidazolinone tolerance to the IMINTA 1, 4, and 5 lines is indicated in bold.

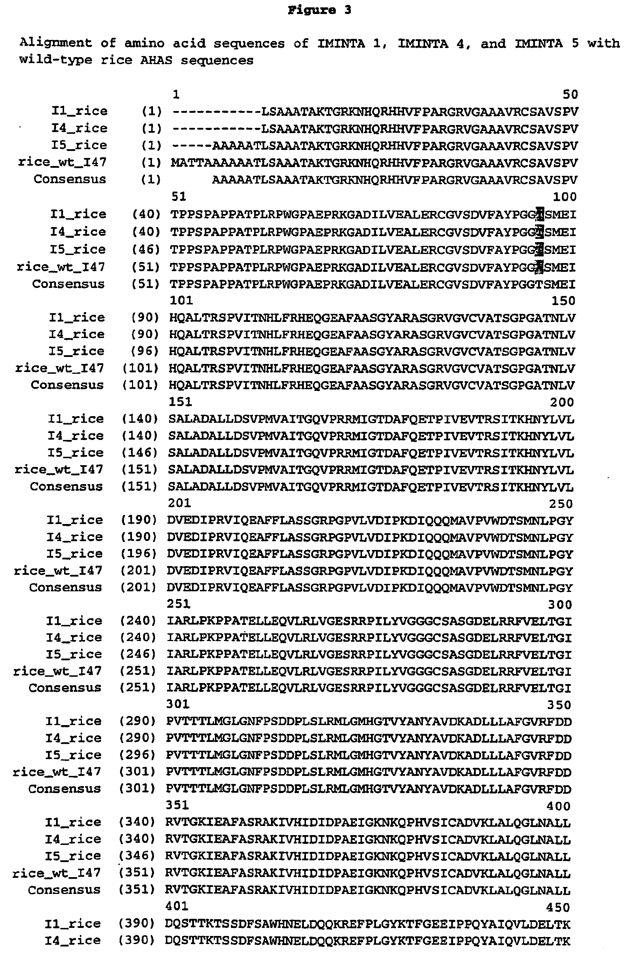

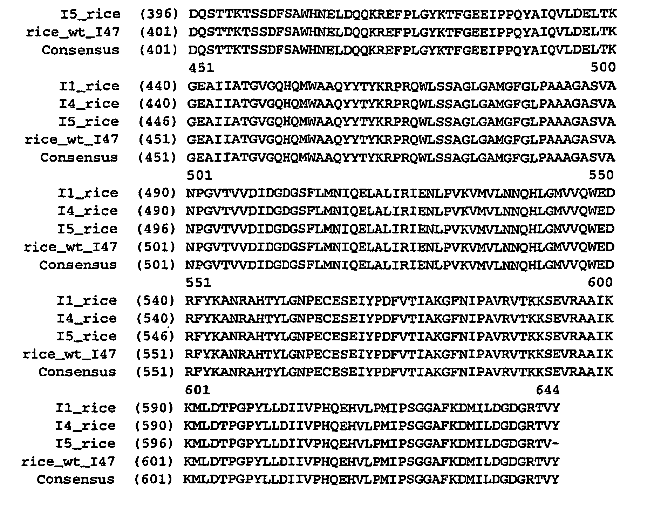

Figure 3 shows the amino acid alignment of the deduced amino acid sequence of the protein encoded by the AHAS gene from the imidazolinone tolerant IMINTA 1 line (SEQ ID NO:2), the deduced amino acid sequence of the protein encoded by the A-HAS gene from the imidazolinone tolerant IMINTA 4 line (SEQ ID NO:4), the deduced amino acid sequence of the protein encoded by the AHAS gene from the imidazolinone tolerant IMINTA 5 line (SEQ ID NO:6), the deduced amino acid sequence of the protein encoded by the AHAS gene from the IRGA 417 wild-type rice line (SEQ ID NO:8), and a rice AHAS amino acid consensus sequence (SEQ ID NO:10). The polymorphism conferring the imidazolinone tolerance to the IMINTA 1, 4, and 5 lines is indicated in bold.
Figure 4A shows an example of a full length cDNA of a variant AHAS nucleic acid (SEQ ID NO:11) and Figure 4B shows an example of the deduced amino acid sequence of the protein encoded by the AHAS gene shown in Figure 4A (SEQ ID NO: 12), wherein the polypeptide confers tolerance to an imidazolinone in comparison to a wild-type AHAS polypeptide.
Figure 5 is a table showing the random block design for the field trial of the IMINTA 1 line and IRGA 417 variety.
Figure 6 is a table showing the response of IRGA 417 and IMINTA 1 lines to treatment by imidazolinone.
Figure 7 is a table showing the grain yield at 14% moisture for IRGA 417 and the IMINTA 1 lines after imidazolinone treatment.
Figure 8 is a table showing the evaluation of the yield components in IRGA 417 and IMINTA 1 lines after imidazolinone treatment.

### Detailed Description

The present invention is directed to rice plants, rice plant parts and rice plant cells having increased tolerance to imidazolinone herbicides. The present invention also includes seeds produced by the rice plants described herein and methods for controlling weeds in the vicinity of the rice plants described herein. It is to be understood that as used in the specification and in the claims, "a" or "an" can mean one or more, depending upon the context in which it is used. Thus, for example, reference to "a cell" can mean that at least one cell can be utilized.

As used herein, the term "rice plant" refers to a plant that is a member of the *Oryza* genus. The rice plants of the present invention can be members of a *Oryza* genus including, but not limited to, *O. alta, O. australiensis, O. barthii, O. brachyantha, O. eichingeri, O. glaberrima, O. glumaepatula, O. grandigiumis, O. granulata, O. latifolia, O. longiglumis, O. longistaminata, O. meridionalis O. meyeriana, O. minuta, O. nivara, O. officinalis, O. punctata, O. rhizomatis, O. ridleyi, O. ruflpogon, O. sativa, and O. schiechteri* and hybrids thereof. Examples of *O. sativa* subspecies included within the present invention are *Japonica,* and *Indica.* A non-limiting cultivar of *Japonica* is Nipponbare, and a non-limiting example of *Indica* is the 93-11 cultivar.

The term -rice plant" is Intended to encompass rice plants at any stage of maturity or development, as well as any tissues or organs (plant parts) taken or derived from any such plant unless otherwise clearly indicated by context. Plant parts include, but are not limited to, stems, roots, flowers, ovules, stamens, leaves, embryos, meristematic regions, callus tissue, anther cultures, gametophytes, sporophytes, pollen, microspores, protoplasts, and the like. The present invention also includes seeds produced by the rice plants of the present invention. In one embodiment, the seeds are true breeding for an increased tolerance to an imidazolinone herbicide as compared to a wild type variety of the rice plant seed.

The present invention describes a rice plant comprising at least one non-recombinant variant AHAS nucleic acid, wherein the rice plant has increased tolerance to an imidazolinone herbicide as compared to a wild-type variety of the plant. As used herein, the term "AHAS gene locus" refers to the position of an AHAS gene on a genome, and the terms AHAS gene" and "AHAS nucleic acid" refer to a nucleic acid encoding the AHAS enzyme.

As used herein, the term "variant AHAS nucleic acid" refers to an AHAS nucleic add having a sequence that is mutated from a wild-type AHAS nucleic acid and that confers increased imidazolinone tolerance to a plant in which it is expressed. As used herein, the term "variant AHAS allele" refers to a single copy of a particular AHAS nucleic acid.

Accordingly, the present invention includes a rice plant comprising a non-recombinant variant AHAS nucleic acid, wherein the rice plant has increased tolerance to an imidazolinone herbicide as compared to a wild-type variety of the plant and wherein the variant AHAS nucleic acid encodes a variant AHAS protein comprising an alanine to threonine mutation as compared to a wild-type AHAS protein. The alanine to threonine mutation corresponds to position 96 of the AHAS amino acid sequence as shown in SEQ ID NO:12. The variant AHAS nucleic acid is selected from the group consisting of a polynucleotide sequence shown in SEQ ID NO: 1; a polynucleotide sequence shown in SEQ ID NO:3; a polynucleotide sequence shown in SEQ ID NO:5; a polynucleotide sequence shown in SEQ ID NO:11; a polynucleotide encoding the polypeptide shown in SEQ ID NO:2; a polynucleotide encoding the polypeptide shown in SEQ ID NO:4; a polynucleotide encoding the polypeptide shown in SEQ ID NO:6; and a polynucleotide encoding the polypeptide shown in SEQ ID NO:12.

The present invention includes rice plants comprising one or more AHAS alleles, wherein the rice plant has increased tolerance to an imidazolinone herbicide as compared to a wild-type variety of the plant. The AHAS alleles can comprise a nucleotide sequence selected from the group consisting of a polynucleotide sequence shown in SEQ ID NO:1; a polynucleotide sequence shown in SEQ ID NO:3; a polynucleotide sequence shown in SEQ ID NO:5; a polynucleotide sequence shown In SEQ ID NO:11; a polynucleotide encoding the polypeptide shown in SEQ ID NO:2; a polynucleotide encoding the polypeptide shown in SEQ ID NO:4; a polynucleotide encoding the polypeptide shown in SEQ ID NO:5; a polynucleotide encoding the polypeptide shown in SEQ ID NO:12; and a polynucleotide complementary to any of the aforementioned polynucleotides.

In one embodiment, the rice plant comprises two different variant AHAS nucleic acids. In another embodiment, the rice plant comprises one variant AHAS nucleic acid, wherein the nucleic acid comprises the polynucleotide sequence of SEQ ID NO:1; SEQ ID NO:3; or SEQ ID NO:5. Preferably, at least one of the variant AHAS nucleic acids comprises a polynucleotide sequence selected from the group consisting of SEQ ID NO:1; SEQ ID NO:3: and SEQ ID NO:5.

The imidazolinone herbicide can be selected from, but is not limited to, PURSUlT® (imazethapyr), CADRE® (imazapic), RAPTOR® (imazamox), SCEPTER® (imazaquin), ASSERT® (imazethabenz), ARSENAL® (imazapyr), a derivative of any of the aforementioned herbicides, or a mixture of two or more of the aforementioned herbicides, for example, imazapyr/imazamox (ODYSSEY®). More specifically, the imidazolinone herbicide can be selected from, but is not limited to, 2-(4-isopropyl-4-methyl-5-oxo-2-imidiazolin-2-yl)-nicotinic acid, 2-(4-isopropyl)-4-methyl-5-oxo-2-imidazolin-2-yl)-3-quinolinecarboxylic acid, 5-ethyl-2-(4-isopropyl-4-methyl-5-oxo-2-imidazolin-2-yl)-nicotinic acid, 2-(4-isopropyl-4-methyl-5-oxo-2-imidazolin-2-yl)-5-(methoxymethyl)-nicotinic acid, 2-(4-isopropyl-4-methyl-5-oxo-2-imidazolin-2-yl)-5-methyinicotinic acid, and a mixture of methyl 6-(4-isopropyl-4-methyl-5-oxo-2-imidazolin-2-yl)-m-toluate and methyl 2-(4-isopropyl-4-methyl-5-oxo-2-imldazolin-2-yl)-p-toluate. The use of 5-ethyl-2-(4-isopropyl-4-methyl-5-oxo-2-imidazolin-2-yl)-nicotinic acid and 2-(4-isopropyl-4-methyl-5-oxo-2-imidazolin-2-yl)-5-(methoxymethyl)-nicotinic acid is preferred. The use of 2-(4-isopropyl-4-methyl-5-oxo-2-imidazolin-2-yl)-5-(methoxymethyl)-nicotinic acid is particularly preferred.

The rice plants described herein can be either transgenic rice plants or non-transgenlc rice plants. As used herein, the term "transgenic" refers to any plant, plant cell, callus, plant tissue, or plant part, that contains all or part of at least one recombinant polynucleotide. In many cases, all or part of the recombinant polynucleotide is stably integrated into a chromosome or stable extra-chromosomal element, so that it is passed on to successive generations. For the purposes of the invention, the term "recombinant polynucleotide refers to a polynucleotide that has been altered, rearranged or modified by genetic engineering. Examples include any cloned polynucleotide, or polynucleotides, that are linked or joined to heterologous sequences. The term "recombinant" does not refer to alterations of polynucleotides that result from naturally occurring events, such as spontaneous mutations, or from non-spontaneous mutagenesis followed by selective breeding. Plants containing mutations arising due to non-spontaneous mutagenesis and selective breeding are referred to herein as non-transgenic plants and are included In the present invention.

An example of a non-transgenic rice plant line comprising one variant AHAS nucleic acid is the plant line deposited with the NCIMB having NCLMB Patent Deposit Designation Number NCLMB 41206, designated herein as the AHAS IMINTA 1 rice line. The partial nucleotide sequence corresponding to the IMINTA 1 AHAS gene is shown in SEQ ID NO:1.

Another example of a non-transgenic rice plant line comprising one AHAS nucleic acid is the plant line deposited with the NCIMB having NCIMB Patent Deposit Designation Number NCIMB 41207, designated herein as the AHAS IMINTA 4 rice line. The partial nucleotide sequence corresponding to the IMINTA 4 AHAS gene is shown in SEQ ID NO:3.

Another example of a non-transgenic rice plant line comprising one AHAS nucleic acid is the plant line deposited with the NCIMB having NCIMB Patent Deposit Designation Number NCIMB 41208, designated herein as the AHAS IMINTA 5 rice line. The partial nucleotide sequence corresponding to the IMINTA 5 AHAS gene is shown in SEQ ID NO:5.

Separate deposits of about 2500 seeds each of the imidazolinone tolerant wheat lines were made with the NCIMB, Aberdeen, Scotland, UK on December 22, 2003. These deposits were made in accordance with the terms and provisions of the Budapest Treaty relating to the deposit of microorganisms. The deposits were made for a term of at least thirty years and at least five years after the most recent request for the fumishing of a sample of the deposit is received by the NCIMB. The deposited seeds were accorded Patent Deposit Designation Numbers NCIMB 41206, NCIMB 41207, and NCIMB 41208.

The present invention includes the rice plant having a Patent Deposit Designation Number NCIMB 41206, NCIMB 41207, or NCIMB 41208; a mutant, recombinant, or genetically engineered derivative of the plant with Patent Deposit Designation Number NCIMB 41208, NCIMB 41207, or NCIMB 41208; any progeny of the plant with Patent Deposit Designation Number NCIMB 41208, NCIMB 41207, or NCIMB 41208; and a plant that is the progeny of any of these plants, in a preferred embodiment, the rice plant of the present invention additionally has the herbicide tolerance characteristics of the plant with Patent Deposit Designation Number NCIMB 41208, NCIMB 41207, and NCIMB 41208.

Also included in the present invention are hybrids of the IMINTA 1, 4, and 5 rice plant lines described herein and hybrids of the IMINTA 1, 4, and 5 with another rice plant.

The terms "cultivar" and "variety" refer to a group of plants within a species defined by the sharing of a common set of characteristics or traits accepted by those skilled in the art as sufficient to distinguish one cultivar or variety from another cultivar or variety. There is no implication in either term that all plants of any given cultivar or variety will be generically identical at either the whole gene or molecular level or that any given plant will be homozygous at all loci. A cultivar or variety is considered "true breeding" for a particular trait if, when the true-breeding cultivar or variety is seif-poltinated, all of the progeny contain the trait. The terms "breeding line" or "line" refer to a group of plants within a cultivar defined by the sharing of a common set of characteristics or traits accepted by those skilled in the art as sufficient to distinguish one breeding line or line from another breeding line or line. There is no implication in either term that all plants of any given breeding line or line will be genetically identical at either the whole gene or molecular level or that any given plant will be homozygous at all loci. A breeding line or line is considered "true breeding" for a particular trait if, when the true-breeding line or breeding line is seif-poitinated, all of the progeny contain the trait. In the present invention, the trait arises from a mutation in an AHAS gene of the rice plant or seed.

It is to be understood that the rice plant of the present invention can comprise a wild-type AHAS nucleic acid in addition to a variant AHAS nucleic acid. As described in Example 2, it is contemplated that the IMINTA 1, 4, and 5 rice lines contain a mutation in only one AHAS allele. Therefore, the present invention includes a rice plant comprising at least one variant AHAS nucleic acid in addition to one or more wild-type AHAS nucleic acids.

In addition to rice plants, isolated AHAS proteins and nucleic acids that preferably confer increased tolerance to an imidazolinone herbicide as compared to wild-type AHAS proteins and nucleic acids are described herein. The isolated AHAS nucleic acids may encode a protein having an alanine to threonine mutation. The alanine to threonine mutation may be located at an amino acid residue corresponding to position 96 of SEQ ID NO: 12. The isolated nucleic acids may comprise a polynucleotide selected from the group consisting of a polynucleotide as defined in SEQ ID NO: 1; a polynucleotide as defined in SEQ ID NO:3; a polynucleotide as defined in SEQ ID NO:5: a polynucleotide as defined in SEQ ID NO:11; a polynucleotide encoding a polypeptide as defined in SEQ ID NO:2; a polynucleotide encoding a polypeptide as defined in SEQ ID NO:4; a polynucleotide encoding a polypeptide as defined in SEQ ID NO:6; a polynucleotide encoding a polypeptide as defined in SEQ ID NO:12; a polynucleotide comprising at least 60 consecutive nucleotides of any of the aforementioned polynucleotides; and a polynucleotide complementary to any of the aforementioned polynucleotides. In a preferred embodiment, the isolated AHAS nucleic acid comprises a polynucleotide sequence of SEQ ID NO:1; SEQ ID NO:3, SEQ ID NO:5 or SEQ ID NO:11.

The term "AHAS protein" or "AHAS polypeptide" refers to an acetohydroxyacid synthase protein, and the terms "variant AHAS protein" or "variant AHAS polypeptide." refers to any AHAS protein that is mutated from a wild-type AHAS protein and that confers increased imidazolinone tolerance to a plant, plant cell, plant part, plant seed, or plant tissue when it is expressed therein. In a preferred embodiment, the variant AHAS protein comprises a polypeptide encoded by a polynucleotide sequence comprising SEQ ID NO:1. in another preferred embodiment, the variant AHAS protein comprises a polypeptide encoded by a polynucleotide sequence comprising SEQ ID NO:3. in another preferred embodiment, the variant AHAS protein comprises a polypeptide encoded by a polynucleotide sequence comprising SEQ ID NO:5. in still another preferred embodiment, the variant AHAS protein comprises a polypeptide comprising SEQ ID NO:2, SEQ ID NO:4 or SEQ ID NO:6.

Also as used herein, the terms "nucleic acid" and "polynucleotide" refer to RNA or DNA that is linear or branched, single or double stranded, or a hybrid thereof. The term also encompasses RNA/DNA hybrids. These terms also encompass untranslated sequence located at both the 3' and 5' ends of the coding region of the gene: at least about 1000 nucleotides of sequence upstream from the 5' end of the coding region and at least about 200 nucleotides of sequence downstream from the 3' end of the coding region of the gene. Less common bases, such as inosine, 5-methylcytosine, 6-methyladenine, hypoxanthine and others can also be used for antisense, dsRNA and ribozyme pairing. For example, polynucleotides that contain C-5 propyne analogues of uridine and cytidine have been shown to bind RNA with high affinity and to be potent antisense inhibitors of gene expression. Other modifications, such as modification to the phosphodiester backbone, or the 2'-hydroxy in the ribose sugar group of the RNA can also be made. The antisense polynucleotides and ribozymes can consist entirely of ribonucleotides, or can contain mixed ribonucleotides and deoxyribonucleotides. The polynucleotides described herein may be produced by any means, including genomic preparations, cDNA preparations, *in vitro* synthesis, RT-PCR and *in vitro* or *in vivo* transcription.

An "isolated" nucleic acid molecule is one that is substantially separated from other nucleic acid molecules, which are present in the natural source of the nucleic acid (i.e., sequences encoding other polypoptides). Preferably, an "isolated" nucleic acid is free of some of the sequences that naturally flank the nucleic acid (i.e., sequences located at the 5' and 3' ends of the nucleic acid) in its naturally occurring replicon. For example, a cloned nuclelc acid is considered isolated. An isolated A-HAS nucleic acid molecule can contain less than about 5 kb, 4 kb, 3 kb, 2 kb,1 kb, 0.5 kb or 0.1 kb of nucleotide sequences which naturally flank the nucleic acid molecule in genomic DNA of the cell from which the nucleic acid is derived (e.g., a *O. sativa* cell). A nucleic acid is also considered isolated if it has been altered by human intervention, or placed in a locus or location that is not its natural site, or if it is introduced into a cell by agroinfection, biolistics, or any other method of plant transformation. Moreover, an "isolated" nucleic acid molecule, such as a cDNA molecule, can be free from some of the other cellular material with which it is naturally associated, or culture medium when produced by recombinant techniques, or chemical precursors or other chemicals when chemically synthesized.

Specifically excluded from the definition of "isolated nucleic acids" are: naturally-occurring chromosomes (such as chromosome spreads), artificial chromosome libraries, genomic libraries, and cDNA libraries that exist either as *an in vitro* nucleic acid preparation or as a transfected/transformed host cell preparation, wherein the host cells are either an *in vitro* heterogeneous preparation or plated as a heterogeneous population of single colonies. Also specifically excluded are the above libraries wherein a specified nucleic acid makes up less than 5% of the number of nucleic add inserts in the vector molecules. Further specifically excluded are whole cell genomic DNA or whole cell RNA preparations (including whole cell preparations that are mechanically sheared or enzymatically digested). Even further specifically excluded are the whole cell preparations found as either an *in vitro* preparation or as a heterogeneous mixture separated by electrophoresis wherein the nucleic acid of the invention has not further been separated from the heterologous nucleic acids in the electrophoresis medium (e.g., further separating by excising a single band from a heterogeneous band population in an agarose gel or nylon blot).

A nucleic acid molecule containing a nucleotide sequence of SEQ ID NO:1, SEQ ID NO:3, SEQ ID NO:5, SEQ ID NO:11 or a portion thereof can be isolated using standard molecular biology techniques and the sequence information provided herein. For example, a. O. *sativa* AHAS cDNA can be isolated from a O. *sativa* library using all or a portion of the sequence of SEQ ID NO:1, SEQ ID NO:3, SEQ ID NO:5 or SEQ ID NO:11. Moreover, a nucleic acid molecule encompassing all or a portion of SEQ ID NO:1, SEQ ID NO:3, SEQ ID NO:5 or SEQ ID NO:11 can be isolated by the polymerase chain reaction using oligonucleotide primers designed based upon this sequence. For example, mRNA can be isolated from plant cells (e.g., by the guanidintum-thiocyanate extraction procedure of Chirgwin et al., 1979, Biochemistry 18:5294-5299), and cDNA can be prepared using reverse transcriptase (e.g., Moloney MLV reverse transcriptase, available from Gibco/BRL, Bethesda, MD; or AMV reverse transcriptase, available from Seikagaku America, inc., St. Petersburg, FL). Synthetic oligonucleotide primers for polymerase chain reaction amplification can be designed based upon the nucleotide sequence shown in SEQ ID NO:1, SEQ ID NO:3, SEQ ID NO:5 or SEQ ID NO:11. A nucleic add molecule of the invention can be amplified using cDNA or, alternatively, genomic DNA, as a template and appropriate oligonucleotide primers according to standard PCR amplification techniques. The nucleic acid molecule so amplified can be cloned into an appropriate vector and characterized by DNA sequence analysis. Furthermore, oligonucleotides corresponding to an AHAS nucleotide sequence can be prepared by standard synthetic techniques, e.g., using an automated DNA synthesizer.

The AHAS nucleic acids described herein can comprise sequences encoding an AHAS protein (i.e., "coding regions"), as well as 5' untranslated sequences and 3' untranslated sequences. Alternatively, the nucleic acid molecules of the present invention can comprise only the coding regions of an AHAS gene, or can contain whole genomic fragments isolated from genomic DNA. A coding region of these sequences is indicated as an "ORF position." Moreover, the nucleic acid molecule of the invention can comprise a portion of a coding region of an AHAS gene, for example, a fragment that can be used as a probe or primer. The nucleotide sequences determined from the cloning of the AHAS genes from *O. sativa* allow for the generation of probes and primers designed for use in identifying and/or cloning AHAS homologs in other cell types and organisms, as well as AHAS homologs from other rice plants and related species. The portion of the coding region can also encode a biologically active fragment of an AHAS protein.

As used herein, the term "biologically active portion of" an AHAS protein is intended to include a portion, e.g., a domain/motif, of an AHAS protein that, when produced in a plant increases the plant's tolerance to an imidazolinone herbicide as compared to a wild-type variety of the plant. Methods for quantitating increased tolerance to imidazolinone herbicides are provided in the Examples below. Biologically active portions of an AHAS protein include peptides derived from SEQ ID NO:2, SEQ ID NO:4, SEQ ID NO:6, or SEQ ID NO:12 which include fewer amino acids than a full length AHAS protein and impart increased tolerance to an imidazolinone herbicide upon expression in a plant. Typically, biologically active portions (e.g., peptides which are, for example, 5, 10, 15, 20, 30, 35, 36, 37, 38, 39, 40, 50, 100, or more amino acids in length) comprise a domain or motif with at least one activity of an AHAS protein. Moreover, other biologically active portions in which other regions of the polypeptide are deleted, can be prepared by recombinant techniques and evaluated for one or more of the activities described herein. Preferably, the biologically active portions of an AHAS protein include one or more conserved domains selected from the group consisting of a Domain A, a Domain B, a Domain C, a Domain D and a Domain E, wherein the conserved domain contains a mutation.

Further, AHAS chimeric or fusion polypeptides are described herein. As used herein, an AHAS "chimeric polypeptide" or "fusion polypeptide" comprises an AHAS polypeptide operatively linked to a non-AHAS polypeptide. A "non-AHAS polypeptide" refers to a polypeptide having an amino acid sequence that is not substantially identical to an AHAS polypeptide, e.g., a polypeptide that is not an AHAS isoenzyme, which peptide performs a different function than an AHAS polypeptide. As used herein with respect to the fusion polypeptide, the term "operatively linked" is intended to indicate that the AHAS polypeptide and the non-AHAS polypeptide are fused to each other so that both sequences fulfill the proposed function attributed to the sequence used. The non-AHAS polypeptide can be fused to the N-torminus or C-terminus of the AHAS polypeptide. For example; in one embodiment, the fusion polypeptide is a GST-AHAS fusion polypeptide in which the AHAS sequence is fused to the C-terminus of the GST sequence. Such fusion polypeptides can facilitate the purification of recombinant AHAS polypeptides, in another embodiment, the fusion polypeptide is an AHAS polypeptide containing a heterologous signal sequence at its N-terminus, in certain host cells (e.g., mammalian host cells), expression and/or secretion of an AHAS polypeptide can be increased through use of a heterologous signal sequence.

An isolated nucleic acid molecule encoding an AHAS polypeptide having a certain percent sequence identity to a polypeptide of SEQ ID NO:2, SEQ ID NO:4, SEQ ID NO:6, or SEQ ID NO:12 can be created by introducing one or more nucleotide substitutions, additions, or deletions into a nucleotide sequence of SEQ ID NO:1, SEQ ID NO:3, SEQ ID NO:5, or SEQ ID NO:11 such that one or more amino acid substitutions, additions, or deletions are introduced into the encoded polypeptide. Mutations can be introduced into a sequence of SEQ ID NO:1, SEQ ID NO:3, SEQ ID NO:5, or SEQ ID NO:11 by standard techniques, such as site-directed mutagenesis and PCR-mediated mutagenesis. Preferably, conservative amino add substitutions are made at one or more predicted non-essential amino acid residues.

A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted nonessential amino acid residue in an AHAS polypeptide is preferably replaced with another amino acid residue from the same side chain family. Alternatively, in another embodiment, mutations can be introduced randomly along all or part of an AHAS coding sequence, such as by saturation mutagenesis, and the resultant mutants can be screened for an AHAS activity described herein to identify mutants that retain AHAS activity. Following mutagenesis of the sequence of SEQ ID NO:1, SEQ ID NO:3, SEQ ID NO:5 or SEQ ID NO:11, the encoded polypeptide can be expressed recombinantly and the activity of the polypeptide can be determined by analyzing the imidazolinone tolerance of a plant expressing the polypeptide as described in the Examples below.

To determine the percent sequence identity of two amino acid sequences, the sequences are aligned for optimal comparison purposes (e.g., gaps can be introduced in the sequence of one polypeptide for optimal alignment with the other polypeptide). The amino acid residues at corresponding amino acid positions are then compared. When a position in one sequence is occupied by the same amino acid residue as the corresponding position in the other sequence, then the molecules are identical at that position. The same type of comparison can be made between two nucleic acid sequences. The percent sequence identity between the two sequences is a function of the number of identical positions shared by the sequences (i.e., percent sequence identity = numbers of identical positions/total numbers of positions x 100). For the purposes of the invention, the percent sequence identity between two nucleic acid or polypeptide sequences is determined using the Vector NTI 6.0 (PC) software package (InforMax, 7600 Wisconsin Ave., Bethesda, MD 20814). A gap opening penalty of 15 and a gap extension penalty of 6.66 are used for determining the percent identity of two nucleic acids. A gap opening penalty of 10 and a gap extension penalty of 0.1 are used for determining the percent identity of two polypeptides. All other parameters are set at the default settings.

It is to be understood that for the purposes of determining sequence identity, when comparing a DNA sequence to an RNA sequence, a thymidine nucleotide equivalent to a uracil nucleotide. Preferably, the isolated AHAS polypeptides described herein are at least about 50-60%, preferably at least about 60-70%, and more preferably at least about 70-75%, 75-80%, 80-85%, 85-90%, or 90-95%, and most preferably at least about 96%, 97%, 98%, 99%, or more identical to an entire amino acid sequence shown in SEQ ID NO:2. SEQ ID NO:4, SEQ ID NO:6, or SEQ ID NO:12

Additionally, optimized AHAS nucleic acids can be created. Preferably, an optimized AHAS nucleic acid encodes an AHAS polypeptide that modulates a plant's tolerance to imidazolinone herbicides, and more preferably increases a plant's tolerance to an imidazolinone herbicide upon its overexpression in the plant. As used herein, "optimized" refers to a nucleic acid that is genetically engineered to increase its expression in a given plant or animal. To provide plant optimized AHAS nucleic acids, the DNA sequence of the gene can be modified to 1) comprise codons preferred by highly ex pressed plant genes; 2) comprise an A+T content in nucleotide base composition to that substantially found in plants; 3) form a plant initiation sequence, 4) eliminate sequences that cause destabilization, inappropriate polyadenylation, degradation and termination of RNA, or that form secondary structure hairpins or RNA splice sites. Increased expression of AHAS nucleic acids in plants can be achieved by utilizing the distribution frequency of codon usage in plants in general or a particular plant. Methods for optimizing nucleic acid expression in plants can be found in EPA 0359472; EPA 0385962; PCT Application No. WO 91/16432; U.S. Patent No. 5,380,831; U.S. Patent No. 5,438,391; Perlack et al., 1991, Prec. Natl. Acad. Sci. USA 88:3324-3328; and Murray et al., 1989, Nucleic Acids Res. 1 7:477498.

As used herein, "frequency of preferred codon usage" refers to the preference exhibited by a specific host cell in usage of nucleotide codons to specify a given amino acid. To determine the frequency of usage of a particular codon in a gene, the number of occurrences of that codon in the gene is divided by the total number of occurrences of all codons specifying the same amino acid in the gene. Similarily, the frequency of preferred codon usage exhibited by a host cell can be calculated by averaging frequency of preferred codon usage in a large number of genes expressed by the host cell. It is preferable that this analysis be limited to genes that are highly expressed by the host cell. The percent deviation of the frequency of preferred codon usage for a synthetic gene from that employed by a host cell is calculated first by determining the percent deviation of the frequency of usage of a single codon from that of the host cell followed by obtaining the average deviation over all codons. As defined herein, this calculation includes unique codons (i.e., ATQ and TGG), in general terms, the overall average deviation of the codon usage of an optimized gene from that of a host cell is calculated using the equation 1A = n = 1 Z Xₙ - YₙXₙ times 100 Z where Xₙ = frequency of usage for codon n in the host cell; Yₙ = frequency of usage for codon n in the synthetic gene, n represents an individual codon that specifies an amino acid and the total number of codons is Z The overall deviation of the frequency of codon usage, A, for all amino acids should preferably be less than about 25%, and more preferably less than about 10%.

Hence, an AHAS nucleic acid can be optimized such that its distribution frequency of codon usage deviates, preferably, no more than 25% from that of highly expressed plant genes and, more preferably, no more than about 10%, in addition, consideration is given to the percentage G+C content of the degenerate third base (monocotyledons appear to favor G+C in this position, whereas dicotyledons do not). It is also recognized that the XCG (where X is A, T, C, or G) nucleotide is the least preferred codon in dicots whereas the XTA codon is avoided in both monocots and dicots. Optimized AHAS nucleic acids of this invention also preferably have CG and TA doublet avoidance indices closely approximating those of the chosen host plant (i.e., *Oryza sativa*)*.* More preferably these indices deviate from that of the host by no more than about 10-1s%.

In addition to the nucleic acid molecules encoding the AHAS polypeptides described above, isolated nucleic acid molecules that are antisense thereto are described herein. Antisense polynucleotides are thought to inhibit gene expression of a target polynucleotide by specifically binding the target polynucleotide and interfering with transcription, splicing, transport, translation and/or stability of the target polynucleotide. Methods are described in the prior art for targeting the antisense polynucleotide to the chromosomal DNA, to a primary RNA transcript or to a processed mRNA. Preferably, the target regions include splice sites, translation initiation codons, translation termination codons, and other sequences within the open reading frame.

The term "antisenso," for the purposes of the invention, refers to a nucleic acid comprising a polynucleotide that is sufficiently complementary to all or a portion of a gene, primary transcript, or processed mRNA, so as to interfere with expression of the endogenous gene. "Complementary" polynucleotides are those that are capable of base pairing according to the standard Watson-Cnck complementarity rules. Specifically, purines will base pair with pyrimidines to form a combination of guanine paired with cytosine (G:C) and adenine paired with either thymine (A:T) in the case of DNA, or adenine paired with uracil (A:U) in the case of RNA. It is understood that two polynucleotides may hybridize to each other even if they are not completely complementary to each other, provided that each has at least one region that is substantially complementary to the other. The term °antlsense nucleic acid" includes single stranded RNA as well as double-stranded DNA expression cassettes that can be transcribed to produce an antisense RNA. "active" antisense nucleic acids are antisense RNA molecules that are capable of selectively hybridizing with a primary transcript or mRNA encoding a polypeptide having at least 80% sequence identity with the polypeptide sequence of SEQ ID NO:2, SEQ ID NO:4, SEQ 10 NO:6, or SEQ ID NO:12.

In addition to the AHAS nucleic acids and polypeptides described above, these nucleic acids and polypeptides attached to a moiety are described herein. These moieties include, but are not limited to, detection moieties, hybridization moieties, purification moieties, delivery moieties, reaction moieties, binding moieties, and the like. A typical group of nucleic acids having moieties attached are probes and primers. Probes and primers typically comprise a substantially isolated oligonucleotide. The oligonucleotide typically comprises a region of nucleotide sequence that hybridizes under stringent conditions to at least about 12, preferably about 25, more preferably about 40, 50, or 75 consecutive nucleotides of a sense strand of the sequence set forth in SEQ ID NO:1, SEQ ID NO:3, SEQ ID NO:5, or SEQ ID NO:11, an anti-sense sequence of the sequence set forth SEQ ID NO:1, SEQ ID NO:3, SEQ ID NO:5, or SEQ ID NO:11, or naturally occurring mutants thereof. Primers based on a nucleotide sequence of sea ID NO:1. SEQ ID NO:3, SEQ ID NO:5, or SEQ ID NO:11 can be used in PCR reactions to clone AHAS homologs. Probes based on the AHAS nucleotide sequences can be used to detect transcripts or genomic sequences encoding the same or homologous polypeptides, in preferred embodiments, the probe further comprises a label group attached thereto, e.g. the label group can be a radioisotope, a fluorescent compound, an enzyme, or an enzyme co-factor. Such probes can be used as a part of a genomic marker test kit for identifying cells which express an AHAS polypeptide, such as by measuring a level of an AHAS-encoding nucleic acid, in a sample of cells, e.g., detecting AHAS mRNA levels or determining whether a genomic AHAS gene has been mutated or deleted.

Further, an isolated recombinant expression vector comprising an AHAS nucleic acid as described above, wherein expression of the vector in a host cell results in increased tolerance to an imidazolinone herbicide as compared to a wild-type variety of host cell is described herein. As used herein, the term "vector" refers to a nucleic acid molecule capable of transporting another nucleic acid to which it has been linked. One type of vector is a "plasmid," which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-eplsomal mammalian vectors) are integrated into the genome of a host cell upon introduction into the host cell, and thereby are replicated along with the host genome. Moreover, certain vectors are capable of directing the expression of genes to which they are operatively linked. Such vectors are referred to herein as "expression vectors." in general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e.g., replication defective retroviruses, adenovlruses, and adeno-assoclated viruses), which serve equivalent functions.

The recombinant expression vectors described herein comprise a nucleic acid of the invention in a form suitable for expression of the nucleic acid in a host cell, which means that the recombinant expression vectors include one or more regulatory sequences, selected on the basis of the host cells to be used for expression, which is operatively linked to the nucleic acid sequence to be expressed. With respect to a recombinant expression vector, "operatively linked" is intended to mean that the nucleotide sequence of interest is linked to the regulatory sequence(s) in a manner which allows for expression of the nucleotide sequence (e.g., in an *in vitro* transcription! translation system or in a host cell when the vector is introduced into the host cell). The term "regulatory sequence" is intended to include promoters, enhancers, and other expression control elements (e.g., polyadenylation signals). Such regulatory sequences are described, for example, in Goeddel, Gene Expression Technology., Methods in Enzymology 185, Academic Press, San Diego, CA (1990) and Gruber and Crosby, in: Methods in Plant Molecular Biology and Biotechnology, Eds. Glick and Thompson, Chapter 7, 89-108, CRC Press: Boca Raton, Florida, including the references therein. Regulatory sequences include those that direct constitutive expression of a nucleotide sequence in many types of host cells and those that direct expression of the nucleotide sequence only in certain host cells or under certain conditions, it will be appreciated by those skilled in the art that the design of the expression vector can depend on such factors as the choice of the host cell to be transformed, the level of expression of polypeptide desired, etc. The expression vectors described herein can be introduced into host cells to thereby produce polypeptides or peptides, including fusion polypeptides or peptides, encoded by nucleic acids as described herein (e.g., AHAS polypeptides, fusion polypeptides, etc.).

The AHAS polypeptides can be expressed in plants and plants cells such as unicellular plant cells (such as algae) (See Faiciatore et al., 1999, Marine Biotechnology 1(3):239-251 and references therein) and plant cells from higher plants (e.g., the spermatophytes, such as crop plants). An AHAS polynucleotide may be "introduced" into a plant cell by any means, including transfection, transformation or transduction, electroporation, particle bombardment, agrolnfection, blollstics and the like.

Suitable methods for transforming or transfecting host cells including plant cells can be found in Sambrook et al. (Molecular Cloning: A Laboratory Manual. 2nd Ed., Cold Spring Harbor Laboratory, Cold Spring Harbor Laboratory Press, Cold Spring Harbor, NY, 1989) and other laboratory manuals such as Methods in Molecular Biology, 1995, Vol. 44, Agrobacterium protocols, Ed: Garland and Davey, Humana Press, Totowa, New Jersey. As increased tolerance to imidazolinone herbicides is a general trait wished to be inherited into a wide variety of plants like maize, wheat, rye, oat, triticale, rice, barley, soybean, peanut, cotton, rapeseed and canola, manihot, pepper, sunflower and tagetes, solanaceous plants like potato, tobacco, eggplant, and tomato, Vicia species, pea, alfalfa, bushy plants (coffee, cacao, tea), Salix species, trees (oll palm, coconut), perennial grasses and forage crops, these crop plants are also preferred target plants for a genetic engineering. Forage crops include, but are not limited limited to, Wheatgrass, Canarygrass, Bromegrass, Wildrye Grass. Bluegrass, Orchardgrass, Alfatfa, Salfoin, Birdsfoot Trefoil, Alsike Clover, Red Clover, and Sweet Clover.

Transfection of an AHAS polynucleotide into a plant can be achieved by *Agrobacterium* mediated gene transfer One transformation method known to those of skill in the art is the , dipping of a flowering plant into an *Agro bacteria* solution, wherein the *Agrobacteria* contains the AHAS nucleic acid., followed by breeding of the transformed gametes. *Agrabacterium* mediated plant transformation can be performed using, for example, the GV3101 (pMP90) (Koncz and Schell,1988, Mol. Gen. Genet. 204:383-398) or LBA4404 (Ctontech) *Agrobacterium tumefaclens* strain. Transformation can be performed by standard transformation and regeneration techniques (Deblaere et al., 1994, Nucl. Acids. Res.13:4777-4788; Galvin, Stanton B. and Schilperoort, Robert A, Plant Molecular Biology Manual, 2nd Ed. - Dordrecht : Kluwer Academic Publ., 1995, - in Sect, Ringbuc Zentrale Signatur: BT11-P ISBN 0-7923-2731-4; Glick, Bernard R. and Thompson, John E., Methods in Planet Molecular Biology and Biotechnology, Boca Raton: CRC Press, 1993 360 S., ISBN 0-8493-5164-2). For example, rapeseed can be transformer via cotyledon or hypocotyl transformation. (Moloney et al., 1989, Plant Cell Report 8:238-242; Do Block et al., 1989, Plant Physiol. 91:694-701). Use of antibiotics for *Agrobacterium* and plant selection depends on the binary vector and the *Agrobacterium* strain used for transformation. Rapeseed selection is normally performed using kanamycin as selectable plant marker. *Agrobacterium* mediated gene transfer to flax can be performed using, for example, a technique described by Mlynarova et al., 1994, Plant Cell Report 13:282-285. Additionally, transformation of soybean can be performed using for example a technique described in European Patent No. 0424 047, U.S. Patent No. 5,322,783, European Patent No. 0397 687, U.S. Patent No. 5,378,543, or U.S. Patent Neo. 5,169,770. Transformation of maize can be achieved by particle bombardment, polyethylene glycol mediated DNA uptake or via the silicon carbide fiber technique. (See, for example, Freeling and Walbot "The maize handbook" Springer Verlag: New York (1993) ISBN 3-540-97828-7). A specific example of maize transformation is found in U.S. Patent No. 5,990,387, and a specific example of wheat transformation can be found in PCT Application No. WO 93/07258.

The introduced AHAS polynudeotide may be maintained in the plant cell stably if it is incorporated into a non-chnomosomal autonomous replicon or integrated into the plant chromosomes. Alternatively, the introduced AHAS polynucleotide may be present on an extra-chromosomal non-replicating vector and be transiently expressed or transtently active. A homologous recombinant microorganism can be created wherein the AHAS polynucleotide is integrated into a chromosome, a vector is prepared which contains at least a portion of an AHAS gene into which a deletion, addition or substitution has been introduced to thereby alter, e.g., functionally disrupt, the endogenous AHAS gene and to create an AHAS gegene. To create a point mutation via homologous recombination, DNA-RNA hybrids can be used in a technique known as chimeraplasty (Cole-Strauss et al.,1899, Nucleic Acids Research 27(5):1323-1330 and Kmiec, 1999, Gene therapy American Scientist 87(3):240-247). Other homologous recombination procedures in *Oryza* species are also well known in the art and are contemplated for use herein.

In the homologous recombination vector, the AHAS gene can be flanked at its 5' and 3' ends by an additional nucleic acid molecule of the AHAS gene to allow for homologous recombination to occur between the exogenous AHAS gene carried by the vector and an endogenous AHAS gene, in a microorganism or plant. The additional flanking AHAS nucleic add molecule is of sufficient length for successful homologous recombination with the endogenous gene. Typically, several hundreds of base pairs up to kilobases of flanking DNA (both at the 5' and 3' ends) are included in the vector (See, e.g., Thomas, K. R., and Capeochi, M. R.,1987, Cell 51:503 for a description of homologous recombination vectors or Strepp at al., 1998, PNAS, 95(8):4368-4373 for cDNA based recombination in *Physcomitrella pstsns*). However, since the AHAS gene normally differs from the AHAS gene at very few amino acids, a flanking sequence is not always necessary. The homologous recombination vector is introduced into a microorganism or plant cell (e.g., via polyethylene glycol mediated DNA), and calls in which the introduced AHAS gene has homologously recombined with the endogenous AHAS gene are selected using art-known techniques

Recombinant microorganisms can be produced that contain selected systems that allow for regulated expression of the introduced gene. For example, inclusion of an AHAS gene on a vector placing it under control of the lac operon permits expression of the AHAS gene only in the presence of IPTG. Such regulatory systems are well known in the art.

Whether present in an extra-chromosomal non-repllcating vector or a vector that is integrated into a chromosome, the AHAS polynucleotide preferably resides in a plant expression cassette. A plant expression cassette preferably contains regulatory sequences capable of driving gene expression in plant cells that are operatively linked so that each sequence can fulfill its function, for example, termination of transcription by polyadenylation signals. Preferred polyadenylation signals are those originating from *Agrobacterium tumefaciens* t-DNA such as the gene 3 known as octopine synthase of the Ti-plasmld pTIACH5 (Glelen et al., 1984, EMBO J. 3:835) or functional equivalents thereof, but also all other terminators functionally active in plants are sultable. As plant gene expression is very often not limited on transcriptional levels, a plant expression cassette preferably contains other operatively linked sequences like translational enhancers such as the overdrive-sequence containing the 5 -untranslated leader sequence from tobacco mosaic virus enhancing the polypeptide per RNA ratio (Gallie et al., 1987, Nucl. Acids Research 15:8693-8711). Examples of plant expression vectors include those detailed in: Becker, D. et al., 1992. New plant binary vectors with selectable markers located proximal to the left border, Plant Mol. Blol. 20:1195-1197; Bevan, M.W., 1984, Binary Agrobacterium vectors for plant transformation, Nucl. Add. Res. 12:8711-8721; and Vectors for Gene Transfer in Higher Plants; in: Transgenic Plants, Vol.1, Engineering and Utillzation, eds.: Kung and R. Wu, Academic Press, 1993. S. 15-38.

Plant gene expression should be operatively linked to an appropriate promoter conferring gene expression in a timely, cell type-preferred, or tissue-preferred manner. Promoters useful in the expression cassettes of the invention include any promoter that is capable of initiating transcription in a plant cell. Such promoters include, but are not limited to those that can be obtained from plants, plant viruses and bacteria that contain genes that are expressed in plants, such as *Agrobacterium* and *Rhizablum.*

The promoter may be constitutive, inducible, developmental stage-preferred, cell type-preferred, tissue-preferred or organ-preferred. Constitutive promoters are active under most conditions. Examples of constitutive promoters include the CaMV 19S and 35S promoters (Odell et al.,1985, Nature 313:810-812), the sX CaMV 35S promoter (Kay et al., 1987, Science 236:1299-1302) the Sep1 promoter, the rice actin promoter (McElroy et al., 1990. Plant Cell 2:163-171), the *Arabidopsis* actin promoter, the ubiquitin promoter (Christensen et al., 1989, Plant Molec. Biol. 18:675-689); pEmu (Last at al.,1991, Theor. Appl. Genet. 81:581-588), the flgwoft mosaic virus 35S promoter, the Smas promoter (Velten et al.,1984, EMBO J. 3:2723-2730), the GRP1-8 promoter, the cinnamyl alcohol dehydrogenase promoter (U.S. Patent No. 5,683,439), promoters from the T-DNA of *Agrobacterium,* such as mannopine synthase, nopaline synthase, and octopine synthase, the small subunit of ribulose biphosphate carboxylase (ssu-RUBISCO) promoter, and the like.

Inducible promoters are active under certain environmental conditions, such as the presence or absence of a nutrient or metabolite, heat or cold, light, pathogen attack, anaerobic conditions, and the like. For example, the hsp80 promoter from *Brassica* is Induced by heat shock; the PPDK promoter is induced by Light; the PR-1 promoter from tobacco, *Arabidopsis,* and maize are inducible by infection with a pathogen; and the Adh1 promoter is induced by hypoxia and cold stress. Plant gene expression can also be facilitated via an inducible promoter (For review, see Gatz, 1997, Annu. Rev. Plant Physlol. Plant Mol. Siol. 48:89-108). Chemically inducible promoters are especially suitable if time-specific gene expression is desired. Examples of such promoters are a salicylic acid inducible promoter (PCT Application No. WO 95/19443), a tetracycline inducible promoter (Gatz et al., 1992, Plant J. 2:397-404) and an ethanol inducible promoter (PCT Application No. WO 93/21334).

Developmental stage-preferred promoters are preferentially expressed at certain stages of development. Tissue and organ preferred promoters include those that are preferentially expressed in certain tissues or organs, such as leaves, roots, seeds, or xylem. Examples of tissue preferred and organ preferred promoters include, but are not limited to fruit-preferred, ovule-preferred, male tissue-preferred, seed-preferred, integument-preferred, tuber-pneffemed, stalk-pmfemd, pericarp-preferred, and leaf-preferred, stigma-preferred, pollen-preferred, anther-preferred, a petal-prefeffed, sepal-preferred, pedicel-preforred, silique-preterred, stem-preferred, root-preferred promoters and the like. Seed preferred promoters are preferentially expressed during seed development and/or germination. For example, seed preferred promoters can be embryo-preferred, endosperm preferred and seed coat-preferred. See Thompson *et al.*, 1989, BloEssays 10:108: Examples of seed preferred promoters include, but are not limited to cellulose synthase (celA), Cim1, gamma-zein, globulln-1, maize 19 kD zeln (cZ19B1) and the like.

Other suitable tissue-preferred or organ-preferred promoters include the napin-gene promoter from rapeseed (U.S. Patent No. 5,608,152), the USP-promoter from *Vicia faba* (Baeumlein et al.,1991, Mol Gen Genet 225(3):459-67), the oleosin-promoter from *Arabidopsis* (PCT Application No. WO 98/45461), the phusolln-promoter from *Phaseolus vulgaris* (U.S. Patent No. 5,504,200), the Bce4-prmoter from Brassica (PCT Application No. WO 91/13980), or the legumin B4 promoter (Le84; Baeumlein et al., 1992, Plant Joumal, 2(2):233-9), as well as promoters conferring seed specific expression in monocot plants like maize, barley, wheat, rye, rice, etc. Suitable promoters to note are the Ipt2 or Ipt1-gene promoter from barley (PCT Application No. WO 95/15389 and PCT Application No. WO 95/23230) or those described in PCT Application No. WO 99/16890 (promoters from the barley hordein-gene, rice glutelin gene, rice oryzin gene, rice prolamin gene, wheat gliadin gene, wheat glutelin gene, oat glutelin gene, Sorghum kaslrin-gene, and rye secalin gene).

Other promoters useful in the expression cassettes described herein include, but are not limited to, the major chlorophyll a/b binding protein promoter, histone promoters, the Ap3 promoter, the β-conglycln promoter, the napin promoter, the soybean lectin promoter, the maize 15kD zein promoter, the 22kD zein promoter, the 27kD zein promoter, the g-zein promoter, the waxy, shrunken 1, shrunken 2, and bronze promoters, the Zm13 promoter (U.S. Patent No. 5,088,169), the maize polygalacturonase promoters (PG) (U.S. Patent Nos. 5,412,085 and 5,545,546), and the SGB6 promoter (U.S. Patent No. 5,470,359), as well as synthetic or other natural promoters.

Additional flexibility in controlling heterologous gene expression in plants may be obtained by using DNA binding domains and response elements from heterologous sources (I.e., DNA binding domains from non-plant sources). An example of such a heterologous DNA binding domain is the LexA DNA binding domain (Brent and Ptashne, 1985, Cell 43:729-738).

Further, host cells into which a herein described recombinant expression vector has been introduced are described herein. The terms "host cell" and "recombinant host ceff are used interchangeably herein. It is understood that such terms refer not only to the particular subject cell but they also apply to the progeny or potential progeny of such a cell. Because certain modifications may occur in succeeding generations due to either mutation or environmental influences, such progeny may not, in fact, be identical to the parent cell, but are still included within the scope of the term as used herein. A host cell can be any prokaryotic or eukeuyotic cell. For example, an AHAS polynucleotide can be expressed in bacterial cells such as *C. glutamicum,* insect cells, fungal cells, or mammalian cells (such as Chinese hamster ovary cells (CHO) or COS cells), algae, ciliates, plant calls, fungi or other microorganisms like *C. glutamicum.* Other suitable host cells are known to those smiled in the art.

A host cell as described herein, such as a prokaryotic or eukaryotic host cell in culture, can be used to produce (i.e., express) an AHAS polynucleotide. Accordingly, methods for producing AHAS polypeptides using the herein described host cells are described herein. The method may comprise culturing the host cell described herein (into which a recombinant expression vector encoding an AHAS polypeptide has been introduced, or into which genome has been introduced a gene encoding a wild-type or AHAS polypeptide) in a suitable medium unti AHAS polypeptide is produced. The method may further comprise isolating AHAS polypeptides from the medium or the host cell. Further isolated AHAS polypeptides, and biologically active portions thereof are described herein. An "isolated" or "purified" poly-peptide or biologically active portion thereof is free of some of the cellular material when produced by recombinant DNA techniques, or chemical precursors or other chemicals when chemically synthesized. The language "substantially free of cellular material" include preparations of AHAS polypeptide in which the polypeptide is separated from some of the cellular components of the cells in which it is naturally or recombinantly produced. The language "substantially free of cellular material" may include preparations of an AHAS polypeptide having less than about 30% (by dry weight) of non-AHAS material (also referred to herein as a "contaminating polypeptides"). more preferably less than about 20% of non-AHAS material, still more preferably less than about 10% of non-AHAS material, and most preferably less than about 5% non-AHAS material.

When the AHAS polypeptide, or biologically active portion thereof, is recombinantly produced, it is also preferably substantially free of culture medium, i.e., culture medium represents less than about 20%, more preferably less than about 10%, and most preferably less than about 5% of the volume of the polypeptide preparation. The language "substantially free of chemical precursors or other chemicals" includes preparations of AHAS polypeptide in which the polypeptide is separated from chemical precursors or other chemicals that are involved in the synthesis of the polypeptide, in one embodiment, the language "substantially free of chemical precursors or other chemicals" includes preparations of an AHAS polypeptide having less than about 30% (by dry weight) of chemical precursors or non-AHAS chemicals, more preferably less than about 20% chemical precursors or non-AHAS chemicals, still more preferably less than about 10% chemical precursors or non-AHAS chemicals, and most preferably less than about 5% chemical precursors or non-AHAS chemicals. In preferred embodiments, isolated polypeptides, or biologically active portions thereof, lack contaminating poly peptides from the same organism from which the AHAS polypeptide is derived. Typically, such polypeptides are produced by recombinant expression of, for example, a *Oryza sativa* AHAS polypeptide in plants other than *Oryza sativa* or microorganisms such as *C. glutamicum,* ciliates, algae, or fungl.

The AHAS polynucleotide and polypeptide sequences described herein have a variety of uses. The nucleic acid and amino acid sequences of the present invention can be used to transform plants, thereby modulating the plant's tolerance to imidazolinone herbicides. Accordingly, a method of producing a transgenic plant having increased tolerance to an imidazolinone herbicide comprising, (a) transforming a plant cell with one or more expression vectors comprising one or more variant AHAS nucleic acids, and (b) generating from the plant call a transgenic plant with an increased tolerance to an imidazolinone herbicide as compared to a wild-type variety of the plant is described herein. The variant AHAS nucleic acid may encode a variant AHAS polypeptide comprising an alanine to threonine mutation as compared to a wild-type AHAS polypeptide.

Further, methods of modifying a plant's tolerance to an imidazolinone herbicide comprising modifying the expression of one or more variant AHAS nucleic acids are described herein. The plant's tolerance to the imidazolinone herbicide can be increased or decreased as achieved by increasing or decreasing the expression of an AHAS polynucleotide, respectively. Preferably, the plant's tolerance to the imidazolinone herbicide is increased by increasing expression of an AHAS polynucleotide. The variant AHAS nucleic acid may encode a variant AHAS polypeptide comprising an alanine to threonine mutation as compared to a wild-type AHAS polypeptide. Expression of an AHAS polynucleotide can be modified by any method known to those of skill in the art. The methods of increasing expression of AHAS polynucleotides can be used wherein the plant is either transgenic or not transgenic. in cases when the plant is transgenic, the plant can be transformed with a vector containing any of the above described AHAS coding nucleic acids, or the plant can be transformed with a promoter that directs expression of endogenous AHAS polynucleotides in the plant, for example. Such a promoter can be tissue specific or developmentally regulated. Alternatively, non-transgenic plants can have endogenous AHAS polynucleotide expression modified by inducing a native promoter. The expression of polynucleotides comprising a polynucleotide sequence as defined ion SEQ ID NO:1, SEQ ID NO:3, SEQ ID NO:5, or SEQ ID NO:11 in target plants can be accomplished by, but is not limited to, one of the following examples: (a) constitutive promoter, (b) chemical-induced promoter, and (c) engineered promoter over-expression with for example zinc-finger derived transcription factors (Greisman & Pabo,1997, Science 275:657).

Transcription of the AHAS polynucleotide can be modulated using zinc-finger derived transcription factors (ZFPs) as described in Grelsman and Pabo, 1997, Science 275:657 and manufactured by Sangamo Biosciences, inc. These ZFPs comprise both a DNA recognition domain and a functional domain that causes activation or repression of a target nucleic acid such as an AHAS nucleic acid. Therefore, activating and repressing ZFPs can be created that specifically recognize the AHAS polynucleotide promoters described above and used to increase or decrease AHAS polynucleotide expression in a plant, thereby modulating the herbicide tolerance of the plant

As desaibed in more detail above, the plants produced by the methods described herein can be monocots or dicots. Tne plants can be selected from maize, wheat, rye, oat, triticale, rice, barley, soybean, peanut, cotton, rapeseed, canola, manihot, pepper, sunflower, tagetes, solanaoeous plants, potato, tobacco, eggplant tomato, Vicia species, pea, atfalfa, coffee, cacao tea, Salix species, oil palm, coconut, perennial grass and forage crops, for example. Forage crops include, but are not limited to, Wheatgrass, Canarygrass, Brome grass, Wildrye Grass. Bluegrass. Orchardgrass, Alfalfa, Salfoin, Birdsfoot Trefoil. Alsike Clover. Red Clover and SWeet Clover. In each of the methods described above, the Plant cell includes, but is not limited to, a protoplast, gamete producing cell, and a cell that regenerates into a whole plant As used herein, the term "transgenic" refers to any plant, plant call, callus, plant tissue, or plant part, that contains all or part of at least one recombinant polynucleotide. In many cases, all or part of the recombinant polynucleotide is stably integrated into a chromosome or stable extra-chromosomal element, so that it is passed on to successive generations.

Tissue culture of various tissues of rices and regeneration of plants therefrom is well known and widely published. For example, reference may be had to Chu, Q. R., et al., (1999) "Use of bridging parents with high anther culturability to improve plant regeneration and breeding value in rice", Rice Biotechnology Quarterly 38:25-28; Chu, Q. R., et al., (1998), "A novel plant regeneration medium for rice anther culture of Southem U.S. crosses", Rice Biotechnology Quarterty 35:15-16; Chu, Q. R., et al., (1997), "A novel basal medium for embryogenic callus induction of Souther US crosses", Rice Biotechnology Quarterly 32:19-20; and Oono, K, "Broadening the Genetic Variability By Tissue Culture Methods', Jap. J. Breed. 33 (Suppl2), 306-307, illus. 1983, the disclosures of which are hereby incorporated herein in their entirety by reference.

Dascribed above are compositions and methods for increasing the imidazolinone tolerance of a rice plant or seed as compared to a wild-type variety of the plant or seed.The imidazolinone tolerance of a rice plant or seed can be increased such that the plant or seed can withstand an imidazolinone herbicide application of preferably approximately 10-400 g ai ha⁻¹, more preferably 20-160 g ai ha⁻¹, and most preferably, 40-80 g ai ha⁻¹. As used herein, to "withstand" an imidazolinone herbicide application means that the plant is either not killed or not injured by such application.

Additionally provided herein is a method of controlling weeds within the vicinity of a rice plant, comprising applying an imidazolinone herbicide to the weeds and to the rice plant, wherein the rice plant has increased tolerance to the imidazolinone herbicide as compared to a wild-type variety of the rice plant and wherein the imidazolinone tolerant rice plant comprises at least one non-recombinant variant AHAS nucleic acid. The variant AHAS nucleic add encodes a variant AHAS polypeptide comprising an alanine to threonine mutation as compared to a wild-type AHAS polypeptide. The mutation is at an amino acid residue corresponding to position 96 of the sequence shown in SEX ID NO:12. By providing for rice plants having increased tolerance to imidazolinone, a wide variety of formulations can be employed for protecting rice plants from weeds, so as to enhance plant growth and reduce competition for nutrients An imidazolinone herbicide can be used by itself for pro-emergence, post-emergence, preplanting, and at-planung control of weeds in areas surrounding the rice plants described herein or an imidazalinone herbicide formulation can be used that contains other additives. The intidazolinone herbicide can also be used as a seed treatment. Additives found in an lmidazolinone herbicide formulation include other herbicides, detorrents, adjuvants, spreading agents, sticking agents, stabilizing agents, or the llike. The imidazolinone herbicide formulation can be a wet or dry preparation and can include, but is not limited to, flowable powders, emulsifiable concentrates and liquid concentrates. The imidazolinone herbicide and herbicide formulations can be applied in accordance with conventional methods, for example, by straying, lrrigation, dusting, or the like.

The invention is further illustrated by the following examples, which are not to be construed in any way as imposing limitations upon the scope thereof. On the contrary, it is to be clearly understood that resort may be had to various other embodiments, modifications, and equivalents thereof, which, after reading the description herein, may suggest themselves to those skilled in the art without departing from the scope of the appended claims.

### EXAMPLES

### Example 1

### Mutagenesis and Selection of imidazolinone Tolerant Rice Lines

Two samples of seeds (600 g each) of the rice cultivar IRGA 417 were treated with a 0.001 M sodium azide aqueous solution at pH 3 (phosphate buffer 0.067M) to produce M1 seed. This treatment was applied by soaking each seed-sample in a two-liter Erlenmeyer containing one liter of the sodium azide sotution, under constant sharing, for 18 hours, at room temperature. After treatment, the seeds were rinsed in tap water and, later on, the seeds were partially dried-aerated on blotting paper sheets in order to extract the moisture from the seeds surface. Afterwards, treated seeds were directly sown at the field nursery.

The M1 seeds were planted in the field nursery with an experimental seed planter Wintersteiger at a rate of 50 plants per square meter. Check lines of a wild-type variety IRGA 417 were planted with push type planter. The lines were grown under flooding conditions until maturity (26% grain moisture) and were bulk harvested. The collected seed (M2) was dried in a convector drier for 14 hours at 45°C. The M2 seeds were kept in close storage until the next season.

The seed from M1 plants (M2 seed) was planted with an experimental seed planter for large areas (AVEC) at a rate of 50 kg/ha. An estimate of 3 ha was finally established comprising a population of 6x10⁶ plants. Check lines of a wild-type variety IRGA 417 were Planted with a push type planter. The entire area was subjected to a selection pressure with a mixture of two lmidazolinone herbicides. Three separate applications of the imidamlinone herbicides were performed with a commercial sprayer in different directions to prevent any escape and resulting in a 3X treatment. A total volume of 222 1/ha was sprayed at 50 psi, with Teejets 8002 nozzles, in each application of imidazoilnone herbicides.

The rate of the 1X treatment was a mixture of Arsenal (imazapyr 75 g a.i/ha) and Cadre (imazapic 24.85 g a.i/ha) in a water solution with a non-ionic surfactant (Citowet) at the rate of 0.25% (v/v). The applications were performed at the four leaf stage of the rice plants. No rainfall was registered during the 7 days after treatments.

Observations were made at regular times to survey the entire area. After 90 days, the surviving individuals were labeled and transplanted to the greenhouse for asexual multiplication and seed production increasing. A total of 10 individual plants were grown, and the seed was harvested and dried in a seed incubator for 7 days at 50 °C. No plants from the check lines survived after the herbicide treatment.

Seed from selected M2 plants were planted in individual pots under greenhouse conditions. A 2X treatment was applied with a backpack R&D Sprayer, divided in two applications of 1X rate of Arsenal (Imazapyr 75 g a.i/ha) and Cadre (Imazapic 24.85 g a.i/ha) in a water solution with a non-ionic surfactant (Citowet) at the rate of 0.25% (v/v). Plants were grown until maturity (26% grain moisture) and hand harvested. The collected seed was subjected to a dormancy breaking treatment of 7 days at 50°C and prepared for a late season planting in the north region.

Seed from the populations of three selected tolerant plants grown in the greenhouse was planted in the field at Las Palmas Chaco for seed increasing. The three populations identified as tolerant to imidazolinone herbicides were named IMINTA 1, IMINTA 4 and IMINTA 5. They were planted with a commercial rice planter at the rate of 50 kg/ha. A treatment of 2X imidazolinone herbicides, Arsenal (Imazapyr 75 g a.i/ha) and Cadre (Imazapic 24 g a.i/ha) in a water solution with a non-ionic surfactant (Citowet) at the rate of 0.25% (v/v) was applied at the four to five leaf stage of the rice plants. No phytotoxic symptoms were observed in any of the three populations. The three populations out yielded the check plot treated with a regular grass herbicide. No segregants were observed and a highly homogenous population either in agronomic and tolerance traits was produced.

### Example 2

### Molecular Characterization of IMINTA 1, IMINTA 4, and IMINTA 5

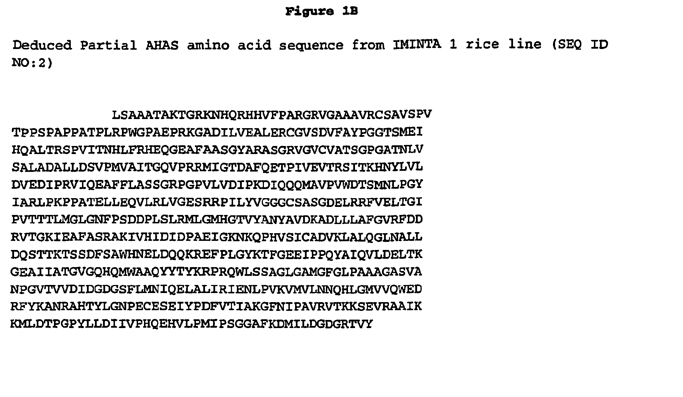

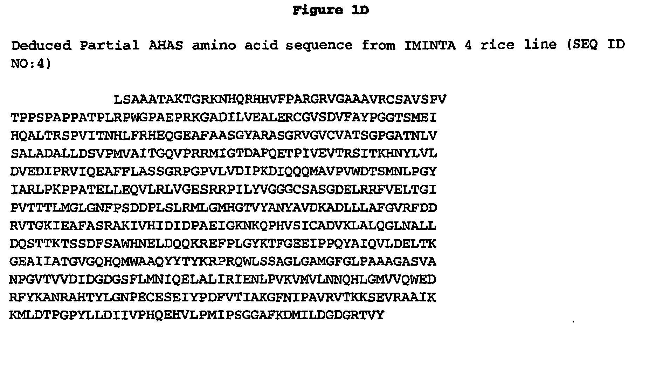

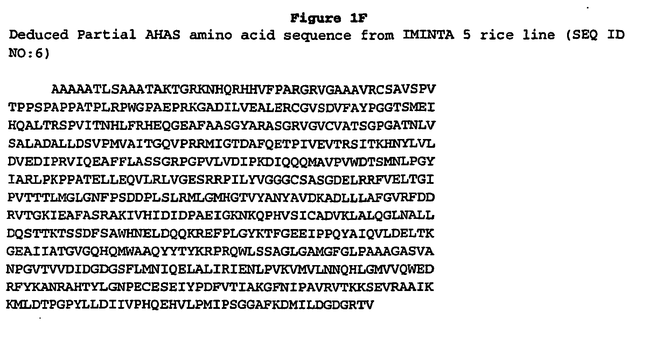

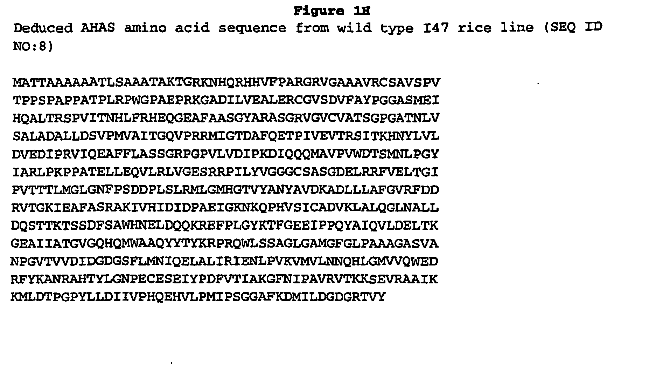

Genomic DNA was extracted from leaves of greenhouse grown seedlings from wild-type and variant IMINTA 1, IMINTA 4, and IMINTA 5 rice lines and the AHAS gene was amplified by PCR. The PCR product was sequenced using standard protocols. Sequence analysis revealed a single base pair change in the coding region of the AHAS gene that caused an amino acid change from Alanine at amino acid 96 in the wild-type line to Threonine 96 in the mutant lines. This mutation corresponds to an amino acid change at Alanine122 in the *Arabidopsis* AHAS sequence to Threonine 122. The AHAS nucleotide sequence for IMINTA 1, IMINTA 4, and IMINTA 5 are shown in Figures 1A, C, and E, respectively as SEQ ID NOs:1, 3, and 5; and the deduced AHAS amino acid sequences of IMINTA 1, IMINTA 4, and IMINTA 5 are shown in Figures 1B, D, and F as SEQ ID NOs:2, 4, and 6, respectively. The nucleotide and deduced amino acid sequences of AHAS from the IRGA 417 wild-type rice strain are shown in Figures 1G and H, respectively, as SEQ ID NOs:7 and 8. Alignments of the AHAS nucleotide and amino acid sequences for IMINTA 1, IMINTA 4, and IMINTA 5 are shown in Figures 2 and Figures 3, respectively. The rice AHAS gene consensus sequence is shown as SEQ ID NO:9, and the deduced amino acid sequence of the rice AHAS consensus sequence is shown as SEQ ID NO:10. The polymorphism conferring the imidazolinone tolerance to the IMINTA 1, 4, and 5 lines is indicated in bold.

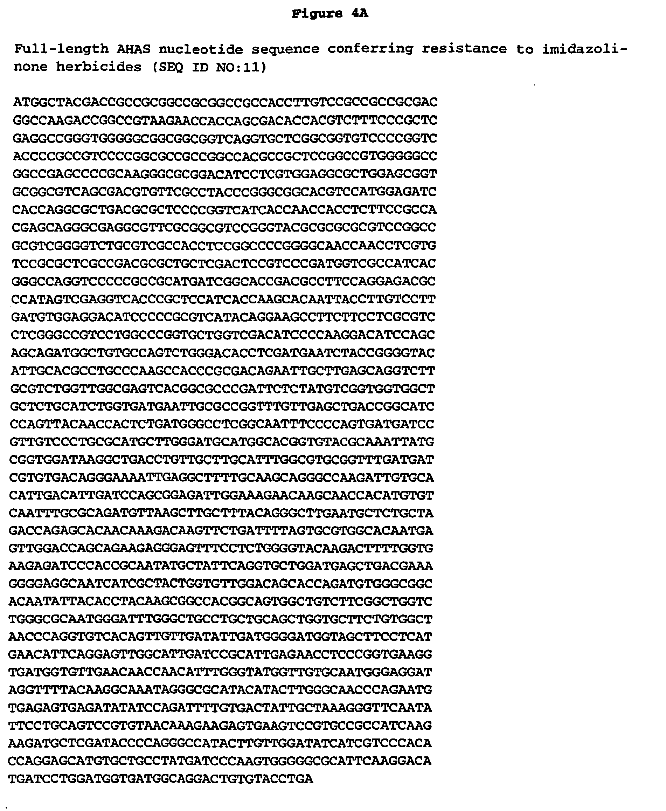

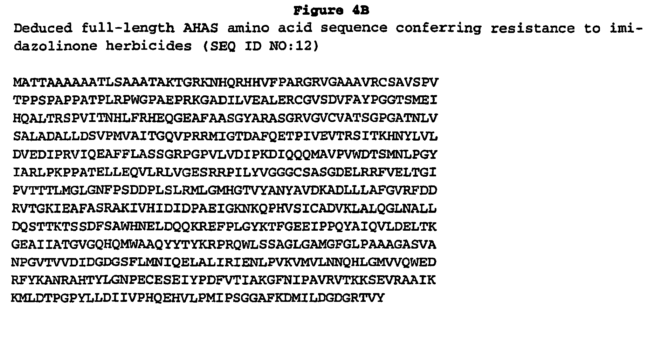

An example of a full length cDNA of an AHAS nucleic acid encoding a polypeptide conferring tolerance to imidazolinone herbicides is shown as SEQ ID NO:11, and the deduced amino acid sequence of the protein encoded by the AHAS gene is shown as SEQ ID NO: 12 in Figure 4.

### Example 3

### Tolerance to AHAS Herbicides Provided by IMINTA 1

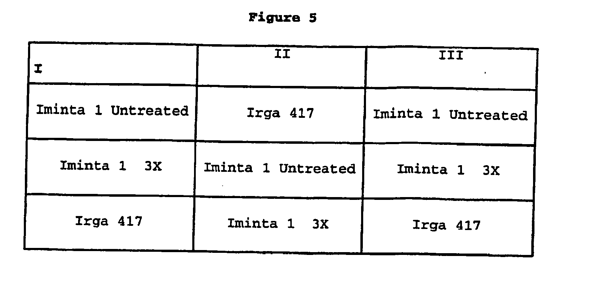

A field trial was performed with the IMINTA 1 mutant line and IRGA 417 line, comparing performance in the presence and absence of imidazolinone treatment. The 1 X imidazolinone treatment consisted of Arsenal (Imazapyr 75 g a.i/ha) and Cadre (Imazapic 24,85 g a.i/ha) in a water solution with a non-ionic surfactant (Citowet) at the rate of 0.25%. The varieties and treatments were set as indicated in Figure 5 as a random block design with three replications.

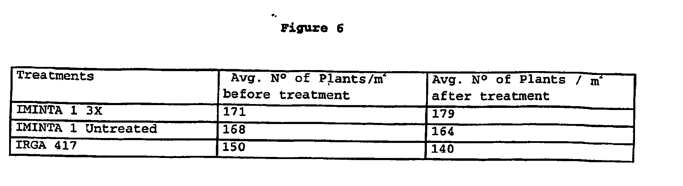

The results of the treatment are set out in Figure 6. There was no statistical difference among treatments in the number of plants/m², thus showing that the 3X herbicide application had no detrimental effect. Susceptible check lines sown along the plots did not survive the herbicide treatment. The higher value in the after treatment IMINTA 3X plots could be due to tillering.

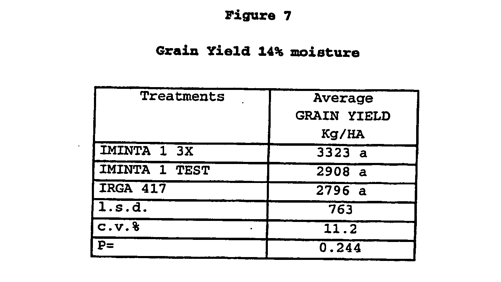

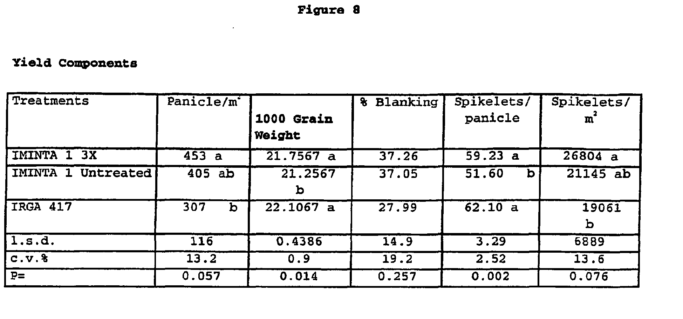

Grain yield and yield components were evaluated to understand the effect of the treatment on the different physiological stages, and are shown in Figures 7 and 8, respectively. No statistical differences were found among treatments, although the absolute values showed a better performance of the IMINTA 1 3X. The analysis of the yield components showed a higher number of panicles per square meter and spike-lets/panicle in the IMINTA 1 3X plots that have determined a higher yield than the other treatments. In addition, a strong blanking percentage was observed due to low temperatures before and during flowering. The cold days and nights reduced the seed set and were the reason for low average yields.

Although there was no statistical differences among treatments, there was a higher grain yield value of the IMINTA 1 3X. As mentioned, more panicles and spikelets/ panicle were found in these plots. Observations on other tolerant lines under higher application treatments showed higher tillering and number of spikelets/panicle suggesting a possible effect of the herbicides on the differentiation process of tillers and flowers.

The IMINTA 4 and IMINTA 5 varieties are also field tested in the same manner as the IMINTA 1 variety. The grain yield and yield components are found to be comparable to the IMINTA 1 variety.

Because the tolerance in IMINTA 1, IMINTA 4, and IMINTA 5 is due to a mutation in the AHAS enzyme rendering it tolerant to inhibition by imidazolinone herbicides, the *in vitro* activity of AHAS extracted from wild-type plants (not having the mutation for tolerance) is compared to the *in vitro* activity of AHAS extracted from tolerant plants in the presence of varying concentrations of an imidazolinone herbicide.

## Claims

1. A rice plant comprising a non-recombinant variant AHAS nucleic acid, wherein the variant AHAS nucleic acid confers upon the plant increased tolerance to an imidazolinone herbicide as compared to a wild-type variety of the plant, wherein the rice plant is a derivative or progeny of the rice plant with Patent Deposit Designation Number NCIMB 41206, NCIMB 41207, or NCIMB 41208, wherein the rice plant with Patent Deposit Designation Number NCIMB 41206, NCIMB 41207, or NCIMB 41208 comprises the variant AHAS nucleic acid, and wherein the variant AHAS nucleic acid comprises a polynucleotide sequence selected from the group consisting of:
(a) a polynucleotide as defined in SEQ ID NO:1, SEQ ID NO:3 or SEQ ID NO:5;
(b) a polynucleotide as defined in SEQ ID NO:11;
(c) a polynucleotide encoding a polypeptide as defined in SEQ ID NO:2, SEQ ID NO:4 or SEQ ID NO:6;
(d) a polynucleotide encoding a polypeptide as defined in SEQ ID NO:12; and
(e) a polynucleotide complementary to the polynucleotide of any of a) through d) above.

2. The rice plant of claim 1, wherein the plant is selected from the group consisting of
(a) a plant that is a mutant, recombinant, or genetically engineered derivative of the plant with Patent Deposit Designation Number NCIMB 41206, NCIMB 41207, or NCRVIB 41208;
(b) any progeny of the plant of Patent Deposit Designation Number NCIMB 41206, NCIMB 41207, or NCIMB 41208; or
(c) a plant that is a progeny of any of the plants of (a) through (b).

3. The rice plant of claim 1 or 2, wherein the variant AHAS nucleic acid comprises a polynucleotide sequence as defined in SEQ ID NO:1.

4. The rice plant of claim 1 or 2, wherein the variant AHAS nucleic acid comprises a polynucleotide sequence as defined in SEQ ID NO:3.

5. The rice plant of claim 1 or 2, wherein the variant AHAS nucleic acid comprises a polynucleotide sequence as defined in SEQ ID NO:5.

6. The rice plant of any one of claims 1 to 5, wherein the imidazolinone herbicide is selected from the group consisting of 2-(4-isopropyl-4-methyl-5-oxo-2-imidazolin-2-yl)-nicotinic acid, 2-(4-isopropyl)-4-methyl-5-oxo-2-imidazolin-2-yl)-3-quinolinecarboxylic acid, 5-ethyl-2-(4-isopropyl-4-methyl-5-oxo-2-imidazolin-2-yl)-nicotinic acid, 2-(4-isopropyl-4-methyl-5-oxo-2-imidazolin-2-yl)-5-(methoxymethyl)-nicotinic acid, 2-(4-isopropyl-4-methyl-5-oxo-2-imidazolin-2-yl)-5-methylnicotinic acid, and a mixture of methyl 6-(4-isopropyl-4-methyl-5-oxo-2-imidazolin-2-yl)-m-toluate and methyl 2-(4-isopropyl-4-methyl-S-oxo-2-imidazolin-2-yl)-p-toluate.

7. The rice plant of any one of claims 1 to 5, wherein the imidazolinone herbicide is 5-ethyl-2-(4-isopropyl-4-methyl-5-oxo-2-imidazolin-2-yl)-nicotinic acid.

8. The rice plant of any one of claims 1 to 5, wherein the imidazolinone herbicide is 2-(4-isopropyl-4-methyl-5-oxo-2-imidazolin-2-yl)-5-(methoxymethyl)-nicotinie acid.

9. A plant part of the rice plant of any one of claims 1 to 8, wherein the plant part comprises the variant AHAS nucleic acid.

10. A plant cell of the rice plant of any one of claims 1 to 8, wherein the plant cell comprises the variant AHAS nucleic acid.

11. A seed produced by the rice plant of any one of claims 1 to 8, wherein the seed comprises the variant AHAS nucleic acid.

12. The seed of claim 11, wherein the seed is true breeding for an increased tolerance to an imidazolinone herbicide as compared to a wild-type variety of the rice plant seed.

13. A method of controlling weeds within the vicinity of a rice plant, comprising applying an imidazolinone herbicide to the weeds and the plant, wherein the plant comprises a non-recombinant variant AHAS nucleic acid which confers upon the plant increased tolerance to the imidazolinone herbicide as compared to a wild-type variety of the plant, wherein the rice plant has Patent Deposit Designation Number NCIMB 41206, NCIMB 41207, or NCIMB 41208 or is a derivative or progeny thereof, wherein the rice plant with Patent Deposit Designation Number NCIMB 41206, NCIMB 41207, or NCIMB 41208 comprises the variant AHAS nucleic acid, and wherein the variant AHAS nucleic acid comprises a polynucleotide sequence selected from the group consisting of:
(a) a polynucleotide as defined in SEQ ID NO:1, SEQ ID NO:3, or SEQ ID NO:5;
(b) a polynucleotide as defined in SEQ ID NO:11;
(c) a polynucleotide encoding a polypeptide as defined in SEQ ID NO:2, SEQ ID NO:4, or SEQ ID NO:6;
(d) a polynucleotide encoding a polypeptide as defined in SEQ ID NO:12; and
(e) a polynucleotide complementary to the polynucleotide of any of a) through d) above.

14. The method of claim 13, wherein the plant is selected from the group consisting of:
(a) a plant having a Patent Deposit Designation Number NCIMB 41206, NCIMB 41207, or NCIMB 41208;
(b) a plant that is a mutant, recombinant, or genetically engineered derivative of the plant with Patent Deposit Designation Number NCIMB 41206, NCIMB 41207, or NCIMB 41208;
(c) any progeny of the plant of Patent Deposit Designation Number NCIMB 41206, NCIMB 41207, or NCIMB 41208; or
(d) a plant that is a progeny of any of the plants of the plants of (a) through (c).

15. The method of claim 13 or 14, wherein the imidazolinone herbicide is selected from the group consisting of 2-(4-isopropyl-4-methyl-5-oxo-2-imidazolin-2-yl)-nicotinic acid, 2-(4-isopropyl)-4-methyl-5-oxo-2-imidazolin-2-yl)-3-quinolinecarboxylic acid, 5-ethyl-2-(4-isopropyl-4-methyl-5-oxo-2-imidazolin-2-yl)-nicotinic acid, 2-(4-isopropyl-4-methyl-5-oxo-2-imidazolin-2-yl)-5-(methoxymethyl)-nicotinic acid, 2-(4-isopropyl-4-methyl-5-oxo-2-imidazolin-2-yl)-5-methylnicotinic acid, and a mixture of methyl 6-(4-isopropyl-4-methyl-5-oxo-2-imidazolin-2-yl)-m-toluate and methyl 2-(4-isopropyl-4-methyl-5-oxo-2-imidazolin-2-yl)-p-toluate.

16. The method of claim 13 or 14, wherein the imidazolinone herbicide is 5-ethyl-2-(4-isopropyl-4-methyl-5-oxo-2-imidazolin-2-yl)-nicotinic acid.

17. The method of claim 13 or 14, wherein the imidazolinone herbicide is 2-(4-isopropyl-4-methyl-5-oxo-2-imidazolin-2-yl)-5-(methoxymethyl)-nicotinic acid.

## Patentansprüche

1. Reispflanze umfassend eine nicht rekombinante AHAS-Nukleinsäurevariante, wobei die AHAS- Nukleinsäurevariante der Pflanze eine im Vergleich zu einer Wildtypvarietät der Pflanze erhöhte Toleranz gegenüber einem Imidazolinon-Herbizid verleiht, wobei die Reispflanze ein Abkömmling oder ein Nachkomme der Reispflanze mit der Patent-Hinterlegungsnummer NCIMB 41206, NCIMB 41207 oder NCIMIB 41208 ist, die Reispflanze mit der Patent-Hinterlegungsnummer NCIMB 41206, NCIMB 41207 oder NCIMIB 41208 die AHAS-Nukleinsäurevariante umfasst und die AHAS-Nukleinsäurevariante eine Polynukleotidsequenz umfasst, die ausgewählt ist unter:
(a) einem wie in SEQ ID NO:1, SEQ ID NO:3 oder SEQ ID NO:5 definierten Polynukleotid,
(b) einem wie in SEQ ID NO:11 definierten Polynukleotid,
(c) einem Polynukleotid, das für ein wie in SEQ ID NO:2, SEQ ID NO:4 oder SEQ ID NO:6 definiertes Polypeptid kodiert,
(d) einem Polynukleotid, das für ein wie in SEQ ID NO:12 definiertes Polypeptid kodiert, und
(e) einem Polynukleotid, das zu einem der oben aufgeführten Polynukleotide (a) bis (d) komplementär ist.

2. Reispflanze nach Anspruch 1, wobei die Pflanze ausgewählt ist unter:
(a) einer Pflanze, die eine Mutante, ein rekombinanter Abkömmling oder ein gentechnisch veränderter Abkömmling der Pflanze mit der Patent-Hinterlegungsnummer NCIMB 41206, NCIMB 41207 oder NCIMIB 41208 ist,
(b) einem Nachkommen der Pflanze mit der Patent-Hinterlegungsnummer NCIMB 41206, NCIMB 41207 oder NCIMIB 41208 oder
(c) einer Pflanze, die ein Nachkomme einer der Pflanzen (a) bis (b) ist.

3. Reispflanze nach Anspruch 1 oder 2, wobei die AHAS-Nukleinsäurevariante eine wie in SEQ ID NO: 1 definierte Polynukleotidsequenz umfasst.

4. Reispflanze nach Anspruch 1 oder 2, wobei die AHAS-Nukleinsäurevariante eine wie in SEQ ID NO:3 definierte Polynukleotidsequenz umfasst.

5. Reispflanze nach Anspruch 1 oder 2, wobei die AHAS-Nukleinsäurevariante eine wie in SEQ ID NO:5 definierte Polynukleotidsequenz umfasst.

6. Reispflanze nach einem der Ansprüche 1 bis 5, wobei das Imidazolinon-Herbizid ausgewählt ist unter 2-(4-Isopropyl-4-methyl-5-oxo-2-imidazolin-2-yl)nicotinsäure, 2-(4-Isopropyl)-4-methyl-5-oxo-2-imidazolin-2-yl)-3-chinolincarbonsäure, 5-Ethyl-2-(4-isopropyl-4-methyl-5-oxo-2-imidazolin-2-yl)nicotinsäure, 2-(4-Isopropyl-4-methyl-5-oxo-2-imidazolin-2-yl)-5-(methoxymethyl)nicotinsäure, 2-(4-Isopropyl-4-methyl-5-oxo-2-imidazolin-2-yl)5-methylnicotinsäure und einem Gemisch aus 6-(4-Isopropyl-4-methyl-5-oxo-2-imidazolin-2-yl)-m-toluylsäuremethylester und 2-(4-Isopropyl-4-methyl-5-oxo-2-imidazolin-2-yl)-p-toluylsäuremethylester.

7. Reispflanze nach einem der Ansprüche 1 bis 5, wobei das Imidazolinon-Herbizid 5-Ethyl-2-(4-isopropyl-4-methyl-5-oxo-2-imidazolin-2-yl)nicotinsäure ist.

8. Reispflanze nach einem der Ansprüche 1 bis 5, wobei das Imidazolinon-Herbizid 2-(4-Isopropyl-4-methyl-5-oxo-2-imidazolin-2-yl)-5-(methoxymethyl)nicotinsäure ist.

9. Pflanzenteil der Reispflanze nach einem der Ansprüche 1 bis 8, wobei der Pflanzenteil die AHAS-Nukleinsäurevariante umfasst.

10. Pflanzenzelle der Reispflanze nach einem der Ansprüche 1 bis 8, wobei die Pflanzenzelle die AHAS-Nukleinsäurevariante umfasst.

11. Von der Reispflanze nach einem der Ansprüche 1 bis 8 ausgebildeter Same, wobei der Same die AHAS-Nukleinsäurevariante umfasst.

12. Same nach Anspruch 11, wobei der Same für eine im Vergleich zu einer Wildtypvarietät der Reispflanze erhöhte Toleranz gegenüber einem Imidazolinon-Herbizid reinerbig ist.

13. Verfahren zur Unkrautbekämpfung in der Nähe einer Reispflanze umfassend das Aufbringen eines Imidazolinon-Herbizids auf das Unkraut und die Pflanze, wobei die Pflanze eine nicht rekombinante AHAS-Nukleinsäurevariante umfasst, die der Pflanze eine im Vergleich zu einer Wildtypvarietät der Pflanze erhöhte Toleranz gegenüber dem Imidazolinon-Herbizid verleiht, die Reispflanze die Patent-Hinterlegungsnummer NCIMB 41206, NCIMB 41207 oder NCIMIB 41208 besitzt oder ein Abkömmling oder ein Nachkomme davon ist, die Reispflanze mit der Patent-Hinterlegungsnummer NCIMB 41206, NCIMB 41207 oder NCIMIB 41208 die AHAS-Nukleinsäurevariante umfasst und die AHAS-Nukleinsäurevariante eine Polynukleotidsequenz umfasst, die ausgewählt ist unter:
(a) einem wie in SEQ ID NO:1, SEQ ID NO:3 oder SEQ ID NO:5 definierten Polynukleotid,
(b) einem wie in SEQ ID NO:11 definierten Polynukleotid,
(c) einem Polynukleotid, das für ein wie in SEQ ID NO:2, SEQ ID NO:4 oder SEQ ID NO:6 definiertes Polypeptid kodiert,
(d) einem Polynukleotid, das für ein wie in SEQ ID NO:12 definiertes Polypeptid kodiert, und
(e) einem Polynukleotid, das zu einem der oben aufgeführten Polynukleotide (a) bis (d) komplementär ist.

14. Verfahren nach Anspruch 13, wobei die Pflanze ausgewählt ist unter:
(a) einer Pflanze mit der Patent-Hinterlegungsnummer NCIMB 41206, NCIMB 41207 oder NCIMIB 41208,
(b) einer Pflanze, die eine Mutante, ein rekombinanter Abkömmling oder ein gentechnisch veränderter Abkömmling der Pflanze mit der Patent-Hinterlegungsnummer NCIMB 41206, NCIMB 41207 oder NCIMIB 41208 ist,
(c) einem Nachkommen der Pflanze mit der Patent-Hinterlegungsnummer NCIMB 41206, NCIMB 41207 oder NCIMIB 41208 oder
(d) einer Pflanze, die ein Nachkomme einer der Pflanzen (a) bis (c) ist.

15. Verfahren nach Anspruch 13 oder 14, wobei das Imidazolinon-Herbizid ausgewählt ist unter 2-(4-Isopropyl-4-methyl-5-oxo-2-imidazolin-2-yl)nicotinsäure, 2-(4-Isopropyl)-4-methyl-5-oxo-2-imidazolin-2-yl)-3-chinolincarbonsäure, 5-Ethyl-2-(4-isopropyl-4-methyl-5-oxo-2-imidazolin-2-yl)nicotinsäure, 2-(4-Isopropyl-4-methyl-5-oxo-2-imidazolin-2-yl)-5-(methoxymethyl)nicotinsäure, 2-(4-Isopropyl-4-methyl-5-oxo-2-imidazolin-2-yl)5-methylnicotinsäure und einem Gemisch aus 6-(4-Isopropyl-4-methyl-5-oxo-2-imidazolin-2-yl)-m-toluylsäuremethylester und 2-(4-Isopropyl-4-methyl-5-oxo-2-imidazolin-2-yl)-p-toluylsäuremethylester.

16. Verfahren nach Anspruch 13 oder 14, wobei das Imidazolinon-Herbizid 5-Ethyl-2-(4-isopropyl-4-methyl-5-oxo-2-imidazolin-2-yl)nicotinsäure ist.

17. Verfahren nach Anspruch 13 oder 14, wobei das Imidazolinon-Herbizid 2-(4-Isopropyl-4-methyl-5-oxo-2-imidazolin-2-yl)-5-(methoxymethyl)nicotinsäure ist.

## Revendications

1. Plante de riz comprenant un variant non recombinant de l'acide nucléique de l'AHAS, dans laquelle le variant de l'acide nucléique de l'AHAS confère à la plante une tolérance accrue à un herbicide imidazolinone, par comparaison avec une variété sauvage de la plante, la plante de riz étant un dérivé ou une descendance de la plante de riz ayant le numéro d'accès NCIMB 41206, NCIMB 41207 ou NCIMB 41208, la plante de riz ayant le numéro d'accès NCIMB 41206, NCIMB 41207 ou NCIMB 41208 comprenant le variant de l'acide nucléique de l'AHAS, et le variant de l'acide nucléique de l'AHAS comprenant une séquence polynucléotidique choisie dans le groupe consistant en :
(a) un polynucléotide tel que défini dans SEQ ID N° 1, SEQ ID N° 3 ou SEQ ID N° 5;
(b) un polynucléotide tel que défini dans SEQ ID N° 11;
(c) un polynucléotide codant pour un polypeptide tel que défini dans SEQ ID N° 2, SEQ ID N° 4 ou SEQ ID N° 6;
(d) un polynucléotide codant pour un polypeptide tel que défini dans SEQ ID N° 12; et
(e) un polynucléotide complémentaire du polynucléotide de l'un quelconque des polynucléotides a) à d) ci-dessus.

2. Plante de riz de la revendication 1, la plante étant choisie dans le groupe consistant en :
(a) une plante qui est un mutant, un recombinant ou un dérivé génétiquement modifié de la plante ayant le numéro d'accès NCIMB 41206, NCIMB 41207 ou NCIMB 41208;
(b) toute descendance de la plante ayant le numéro d'accès NCIMB 41206, NCIMB 41207 ou NCIMB 41208; ou
(c) une plante qui est une descendance de l'une quelconque des plantes (a) à (b).

3. Plante de riz de la revendication 1 ou 2, dans laquelle le variant de l'acide nucléique de l'AHAS comprend une séquence polynucléotidique telle que définie dans SEQ ID N° 1.

4. Plante de riz de la revendication 1 ou 2, dans laquelle le variant de l'acide nucléique de l'AHAS comprend une séquence polynucléotidique telle que définie dans SEQ ID N° 3.

5. Plante de riz selon la revendication 1 ou 2, dans laquelle le variant de l'acide nucléique de l'AHAS comprend une séquence polynucléotidique telle que définie dans SEQ ID N° 5.

6. Plante de riz de l'une quelconque des revendications 1 à 5, dans laquelle l'herbicide imidazolinone est choisi dans le groupe consistant en l'acide 2-(4-isopropyl-4-méthyl-5-oxo-2-imidazolin-2-yl)-nicotinique, l'acide 2-(4-isopropyl)-4-méthyl-5-oxo-2-imidazolin-2-yl)-3-quinoléinecarboxylique, l'acide 5-éthyl-2-(4-isopropyl-4-méthyl-5-oxo-2-imidazolin-2-yl)-nicotinique, l'acide 2-(4-isopropyl-4-méthyl-5-oxo-2-imidazolin-2-yl)-5-(méthoxyméthyl)-nicotinique, l'acide 2-(4-isopropyl-4-méthyl-5-oxo-2-imidazolin-2-yl)-5-méthylnicotinique, et un mélange de 6-(4-isopropyl-4-méthyl-5-oxo-2-imidazolin-2-yl)-m-toluate de méthyle et de 2-(4-isopropyl-4-méthyl-5-oxo-2-imidazolin-2-yl)-p-toluate de méthyle.

7. Plante de riz de l'une quelconque des revendications 1 à 5, dans laquelle l'herbicide imidazolinone est l'acide 5-éthyl-2-(4-isopropyl-4-méthyl-5-oxo-2-imidazolin-2-yl)-nicotinique.

8. Plante de riz de l'une quelconque des revendications 1 à 5, dans laquelle l'herbicide imidazolinone est l'acide 2-(4-isopropyl-4-méthyl-5-oxo-2-imidazolin-2-yl)-5-(méthoxyméthyl)-nicotinique.

9. Partie végétale de la plante de riz de l'une quelconque des revendications 1 à 8, la partie végétale comprenant le variant de l'acide nucléique AHAS.

10. Cellule végétale de la plante de riz de l'une quelconque des revendications 1 à 8, la cellule végétale comprenant le variant de l'acide nucléique AHAS.

11. Graine produite par la plante de riz de l'une quelconque des revendications 1 à 8, la graine comprenant le variant de l'acide nucléique AHAS.

12. Graine de la revendication 11, la graine étant une lignée pure pour ce qui est d'une tolérance accrue à un herbicide imidazolinone, par comparaison avec une variété sauvage de la graine de plante de riz.

13. Procédé pour maîtriser les mauvaises herbes au voisinage d'une plante de riz, comprenant l'application d'un herbicide imidazolinone sur les mauvaises herbes et sur la plante, la plante comprenant un variant non recombinant de l'acide nucléique de l'AHAS, qui confère à la plante une tolérance accrue à l'herbicide imidazolinone par comparaison avec une variété sauvage de la plante, la plante de riz ayant le numéro d'accès NCIMB 41206, NCIMB 41207, ou NCIMB 41208, ou en est un dérivé ou une descendance, la plante de riz ayant le numéro d'accès NCIMB 41206, NCIMB 41207 ou NCIMB 41208 comprenant le variant de l'acide nucléique de l'AHAS, et le variant de l'acide nucléique de l'AHAS comprenant une séquence polynucléotidique choisie dans le groupe consistant en :
(a) un polynucléotide tel que défini dans SEQ ID N° 1, SEQ ID N° 3 ou SEQ ID N° 5;
(b) un polynucléotide tel que défini dans SEQ ID N° 11;
(c) un polynucléotide codant pour un polypeptide tel que défini dans SEQ ID N° 2, SEQ ID N° 4 ou SEQ ID N° 6;
(d) un polynucléotide codant pour un polypeptide tel que défini dans SEQ ID N° 12; et
(e) un polynucléotide complémentaire du polynucléotide de l'un quelconque des polynucléotides a) à d) ci-dessus.

14. Procédé de la revendication 13, dans lequel la plante est choisie dans le groupe consistant en :
(a) une plante ayant un numéro d'accès NCIMB 41206, NCIMB 41207 ou NCIMB 41208;
(b) une plante qui est un mutant, un recombinant ou un dérivé génétiquement modifié de la plante ayant le numéro d'accès NCIMB 41206, NCIMB 41207 ou NCIMB 41208;
(c) toute descendance de la plante ayant le numéro d'accès NCIMB 41206, NCIMB 41207 ou NCIMB 41208; ou
(d) une plante qui est une descendance de l'une quelconque des plantes (a) à (c).

15. Procédé de la revendication 13 ou 14, dans lequel l'herbicide imidazolinone est choisi dans le groupe consistant en l'acide 2-(4-isopropyl-4-méthyl-5-oxo-2-imidazolin-2-yl)-nicotinique, l'acide 2-(4-isopropyl)-4-méthyl-5-oxo-2-imidazolin-2-yl)-3-quinoléine-carboxylique, l'acide 5-éthyl-2-(4-isopropyl-4-méthyl-5-oxo-2-imidazolin-2-yl)-nicotinique, l'acide 2-(4-isopropyl-4-méthyl-5-oxo-2-imidazolin-2-yl)-5-(méthoxyméthyl)-nicotinique, l'acide 2-(4-isopropyl-4-méthyl-5-oxo-2-imidazolin-2-yl)-5-méthylnicotinique, et un mélange de 6-(4-isopropyl-4-méthyl-5-oxo-2-imidazolin-2-yl)-m-toluate de méthyle et de 2-(4-isopropyl-4-méthyl-5-oxo-2-imidazolin-2-yl)-p-toluate de méthyle.

16. Procédé de la revendication 13 ou 14, dans lequel l'herbicide imidazolinone est l'acide 5-éthyl-2-(4-isopropyl-4-méthyl-5-oxo-2-imidazolin-2-yl)-nicotinique.

17. Procédé de la revendication 13 ou 14, dans lequel l'herbicide imidazolinone est l'acide 2-(4-isopropyl-4-méthyl-5-oxo-2-imidazolin-2-yl)-5-(méthoxyméthyl)-nicotinique.
